# EUROPEAN PATENT SPECIFICATION

(11) **EP 2 596 134 B1**
(45) Date of publication and mention of the grant of the patent: **08.04.2020**
(21) Application number: 11810464.5
(22) Date of filing: 22.07.2011
(51) Int. Cl.: C12Q 1/68, G01N 33/53

(54) **METHODS OF DETECTING DISEASES OR CONDITIONS USING PHAGOCYTIC CELLS**
VERFAHREN ZUR ERKENNUNG VON ERKRANKUNGEN ODER LEIDEN MIT PHAGOZYTISCHEN ZELLEN
MÉTHODES DE DÉPISTAGE DE MALADIES OU D'AFFECTIONS À L'AIDE DE CELLULES PHAGOCYTAIRES

(30) Priority: 23.07.2010 US 367094 P
(43) Date of publication of application: 29.05.2013
(73) Proprietor: President and Fellows of Harvard College, Cambridge, MA 02138 (US)
(72) Inventor: KASSIS, Amin, I., Chestnut Hill Massachusetts 02467 (US)
(74) Representative: Barker Brettell LLP
(86) International application number: PCT/US2011/045018
(87) International publication number: WO 2012/012725

(56) References cited:
- EP-A1- 2 161 577
- WO-A1-2009/092068
- WO-A1-2009/092068
- WO-A2-2005/020784
- US-A1- 2004 265 932
- US-A1- 2010 056 523
- US-A1- 2010 184 031
- US-A1- 2011 033 839
- Math P.G. Leers ET AL: "Circulating PSA-Containing Macrophages as a Possible Target for the Detection of Prostate Cancer", AMERICAN JOURNAL OF CLINICAL PATHOLOGY, vol. 129, no. 4, 1 April 2008 (2008-04-01) , pages 649-656, XP055249226, US ISSN: 0002-9173, DOI: 10.1309/THWWRU8L42U5H9PB

## Description

### Field of the Invention

This invention relates generally to methods of using phagocytic cells in the diagnosis, prognosis, or monitoring of a disease or condition. The invention also relates to methods of using phagocytic cells to identify markers of diseases or conditions.

### Background of the Invention

Early diagnosis of a disease often increases the likelihood of successful treatment or cure of such disease. Current diagnostic methods, however, depend largely on population-derived average values obtained from healthy individuals. Personalized diagnostic methods are needed that enable the diagnosis, especially the early diagnosis, of the presence of a disease or a condition in individuals who are not known to have the disease or who have recurrent disease.

Leukocytes begin as pluripotent hematopoietic stem cells in the bone marrow and develop along either the myeloid lineage (monocytes, macrophages, neutrophils, eosinophils, and basophils) or the lymphoid lineage (T and B lymphocytes and natural killer cells). The major function of the myeloid lineage cells (e.g., neutrophils and macrophages) is the phagocytosis of infectious organisms, live unwanted damaged cells, senescent and dead cells (apoptotic and necrotic), as well as the clearing of cellular debris. Phagocytes from healthy animals do not replicate and are diploid, i.e., have a DNA index of one. On average, each cell contains <10 ng DNA, <20 ng NA, and <300 ng of protein.

One object is to provide diagnostic methods that can facilitate the detection of a disease or condition-specific markers, e.g., nucleic acids, proteins, carbohydrates, and/or lipids and the like by using phagocytic cells. Another object is to provide methods of identifying a disease or condition-specific markers and further use such markers alone or together with any known markers to diagnose diseases or conditions.

### Summary of the Invention

We describe new and useful methods for detecting/diagnosing diseases or conditions by using phagocytic cells having different levels of DNA contents (i.e., >2n and =2n). In some descriptions, two sub-populations of phagocytic cells are used, wherein the phagocytic cells that have a DNA content greater than 2n (the >2n phagocytic cells) serve as surrogates for diseased cells, while the phagocytic cells that have a DNA content of 2n (the =2n phagocytic cells) serve as control cells.

According to one aspect of the present invention, there is provided a method for monitoring the progression or regression of cancer in a subject in accordance with claim 1 herein.

Also described is a method for diagnosing or aiding in the diagnosis of a disease or condition in a subject comprising: a) determining a first profile of one or more markers of the disease or condition from a population of phagocytic cells having a DNA content more than 2n (>2n phagocytic cells); b) determining a second profile of at least one of the one or more markers from a population of phagocytic cells having a DNA content of 2n (=2n phagocytic cells); and c) identifying a difference between the first and second profiles of at least one or more of said markers, wherein the difference is indicative of the presence of said disease or condition in the subject.

Also described is a method for assessing the risk of developing a disease or condition in a subject comprising: a) determining a first profile of one or more markers of the disease or condition from a population of >2n phagocytic cells; b) determining a second profile of at least one of the one or more markers from a population of =2n phagocytic cells; and c) identifying a difference between the first and second profiles of at least one or more of said markers, wherein the difference is indicative of the risk of developing said disease or condition in the subject.

Also described is a method for prognosing or aiding in the prognosis of a disease or condition in a subject comprising: a) determining a first profile of one or more markers of the disease or condition from a population of >2n phagocytic cells; b) determining a second profile of at least one of the one or more markers from a population of =2n phagocytic cells; and c) identifying a difference between the first and second profiles of at least one or more of said markers, wherein the difference is indicative of the prognosis of said disease or condition in the subject.

Also described is a method for assessing the efficacy of a treatment for a disease or condition in a subject comprising: a) determining a first profile of one or more markers of the disease or condition from a population of >2n phagocytic cells from the subject before the treatment; determining a second profile of at least one of the one or more markers from a population of =2n phagocytic cells from the subject before the treatment; identifying a first difference between the first and second profiles of at least one or more of said markers; b) determining a third profile of the one or more markers from a population of >2n phagocytic cells from the subject after the treatment; determining a fourth profile of at least one of the one or more markers from a population of =2n phagocytic cells from the subject after the treatment; identifying a second difference between the third and fourth profiles of at least one or more of said markers; and c) identifying a difference between the first difference and the second difference, wherein the identified difference is indicative of the efficacy of the treatment for said disease or condition in the subject.

Also described is a method for monitoring the progression or regression of a disease or condition in a subject comprising: a) determining a first profile of one or more markers of the disease or condition from a population of >2n phagocytic cells from the subject at a first time point; determining a second profile of at least one of the one or more markers from a population of =2n phagocytic cells from the subject at the first time point; identifying a first difference between the first and second profiles of at least one or more of said markers; b) determining a third profile of the one or more markers from a population of >2n phagocytic cells from the subject at a second time point; determining a fourth profile of at least one of the one or more markers from a population of =2n phagocytic cells from the subject at the second time point; identifying a second difference between the third and fourth profiles of at least one or more of said markers; and c) identifying a difference between the first difference and the second difference, wherein the identified difference is indicative of the progression or regression of said disease or condition in the subject.

Also described is a method for identifying a compound capable of ameliorating or treating a disease or condition in a subject comprising: a) determining a first profile of one or more markers of the disease or condition from a population of >2n phagocytic cells from the subject before administering the compound to the subject; determining a second profile of at least one of the one or more markers from a population of =2n phagocytic cells from the subject before administering the compound to the subject; identifying a first difference between the first and second profiles of at least one or more of said markers; b) determining a third profile of the one or more markers from a population of >2n phagocytic cells from the subject after the administration of the compound; determining a fourth profile of at least one of the one or more markers from a population of =2n phagocytic cells from the subject after the administration of the compound; identifying a second difference between the third and fourth profiles of at least one or more of said markers; c) identifying a difference between the first difference and the second difference, wherein the identified difference indicates that the compound is capable of ameliorating or treating said disease or condition in the subject.

Also described is a method for identifying one or more markers of a disease or condition comprising: a) determining a first profile of analytes from >2n phagocytic cells from a subject having said disease or condition; determining a second profile of analytes from =2n phagocytic cells from the subject having said disease or condition; identifying a first set of differences between the first and second profiles, wherein the first set of differences is specific to the first profile relative to the second profile; b) determining a third profile of analytes from >2n phagocytic cells from a control subject not having said disease or condition; determining a fourth profile of analytes from =2n phagocytic cells from the control subject not having said disease or condition; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises: d) obtaining a fifth profile of analytes from cells or tissues affected by said disease or condition from the subject having said disease or condition; obtaining a sixth profile of analytes from cells or tissues not affected by said disease or condition from the subject having said disease or condition; identifying a third set of differences between the fifth and sixth profiles, wherein the third set of differences is specific to the fifth profile relative to the sixth profile; and e) identifying at least one of the one or more markers of c) present in the third set of differences .

In some descriptions, the markers or the analytes are nucleic acids (e.g., nucleotides, oligonucleotides, DNAs, RNAs, or DNA-RNA hybrids), proteins (e.g., , amino acids, peptides, enzymes, antigens, antibodies, cytokines, lipoproteins, glycoproteins, or hormones), lipids (e.g., fatty acids, phosphatides, cholesterol), carbohydrates (e.g., monosaccharides, disaccharides, polysaccharides), metabolites (e.g., vitamins, primary metabolites, secondary metabolites), or combinations thereof.

In some descriptions, the profile is a nucleic acid profile (e.g., genotypic profile, a single nucleotide polymorphism profile, a gene mutation profile, a gene copy number profile, a DNA methylation profile, a DNA acetylation profile, a chromosome dosage profile, a gene expression profile), a protein profile (e.g., protein expression, protein activation), a lipid profile, a carbohydrate profile, a metabolite profile, or a combination thereof. In some descriptions, the profile is determined by a qualitative assay, a quantitative assay, or a combination thereof.

In some descriptions, at least one of the one or more markers is up- regulated or activated in the >2n phagocytic cells compared to the =2n phagocytic cells. In some descriptions, at least one of the one or more markers is down- regulated or inhibited in the >2n phagocytic cells compared to the =2n phagocytic cells.

In some descriptions, the first profile, the second profile, the third profile, the fourth profile, the fifth profile, or the sixth profile comprises the absence of at least one of the one or more markers.

In some descriptions, the difference is at least 1.05-fold, 1.1 -fold, 1.2- fold, 1.3-fold, 1.4-fold, 1.5-fold, 2-fold, 2.5-fold, 3-fold, 4-fold, 5-fold, 6-fold, 7- fold, 8-fold, 9-fold, or 10-fold difference.

In some descriptions, the methods described also comprise lysing the phagocytic cells (e.g., >2n phagocytic cells, or =2n phagocytic cells) and also extracting the cellular contents from those cells. In some descriptions, the cellular contents of the >2n phagocytic cells comprise viable diseased cells, dead diseased cells, apoptotic diseased cells, circulating tumor cells, infectious agents, fetal cells, trophob lasts, or fragments thereof.

In some descriptions, at least one of the one or more markers of the disease or condition is present in the cellular contents of the >2n phagocytic cells. In some descriptions, the one or more markers of said disease or condition are not present in the cellular contents of the =2n phagocytic cells. In some descriptions, the phagocytic cells express at least one of the one or more markers of said disease or condition. In some descriptions, the >2n phagocytic cells express at least one of the one or more markers of said disease or condition.

In some descriptions, the methods also comprise comparing the identified difference of c) to a repository of one or more known markers of said disease or condition (e.g., data obtained by data mining).

In some descriptions, the phagocytic cells are professional phagocytic cells (e.g., neutrophils, macrophages, monocytes, dendritic cells, foam cells, mast cells, eosinophils), non-professional phagocytic cells (e.g., epithelial cells, endothelial cells, fibroblasts, mesenchymal cells), or mixtures thereof.

In some descriptions, the phagocytic cells (e.g., >2n phagocytic cells, =2n phagocytic cells) are isolated from a bodily fluid sample (e.g., blood, urine), tissues, or cells (e.g., white blood cells, fetal cells) of the subject. In some descriptions, the >2n phagocytic cells and the =2n phagocytic cells are isolated from a population of phagocytic cells.

In some descriptions, a standard/know cell separation/isolation/purification technique, such as antibody, flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platforms, or a combination thereof, is used to isolate phagocytic cells (e.g., >2n phagocytic cells and =2n phagocytic cells) from bodily fluids, tissues or cells, or to separate >2n phagocytic cells from =2n phagocytic cells. In some descriptions, the >2n phagocytic cells can also be isolated by using a product secreted by the >2n phagocytic cells, or by using a cell surface target (e.g., a receptor protein, a marker of said disease or condition) on the surface of the >2n phagocytic cells. In some descriptions, the target is expressed by the >2n phagocytic cells. In other descriptions, the target is not expressed by the >2n phagocytic cells. In some descriptions, the >2n phagocytic cells and the =2n phagocytic cells are isolated using a ligand that binds to a molecular receptor expressed on the plasma membranes of white blood cells.

Also described are markers that can be used in the methods described herein and that can be identified by the methods described herein.

### Brief Description of the Drawings

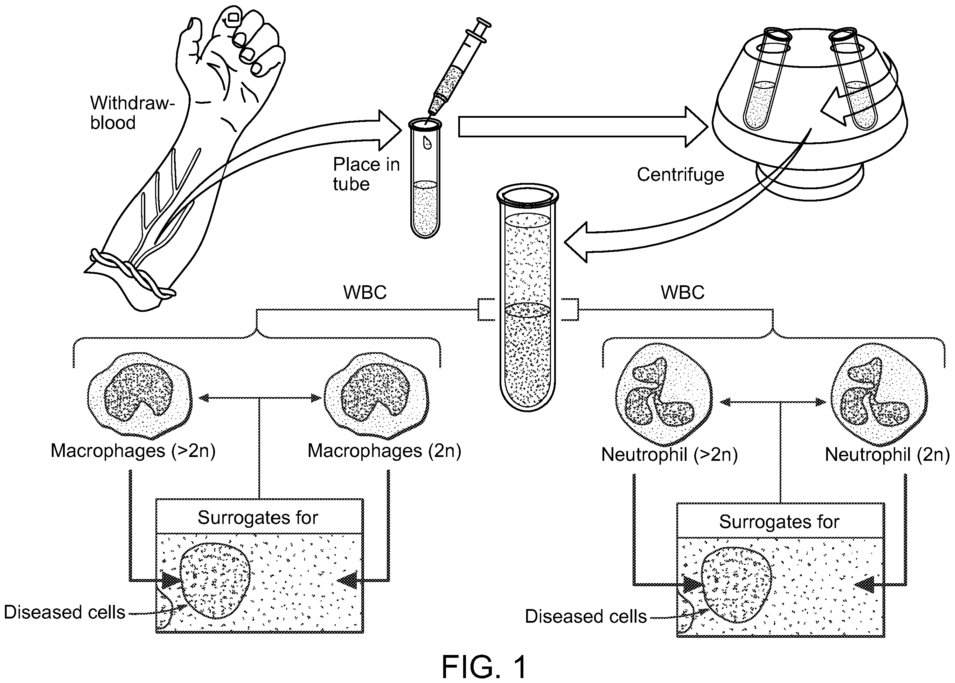

**FIG. 1** schematically depicts one description of a method described herein for diagnosing or aiding in the diagnosis of a disease or condition. In this description, a blood sample is withdrawn from an individual to be diagnosed. After a centrifugation step, white blood cells are isolated from the blood sample and further separated into two populations of phagocytic cells: phagocytic cells (e.g., macrophages or neutrophils) having a DNA content more than 2n (>2n phagocytic cells) and phagocytic cells (e.g., macrophages or neutrophils) having a DNA content of 2n (=2n phagocytic cells). The >2n phagocytic cells serve as surrogates for diseased cells and the 2n phagocytic cells serve as control cells.

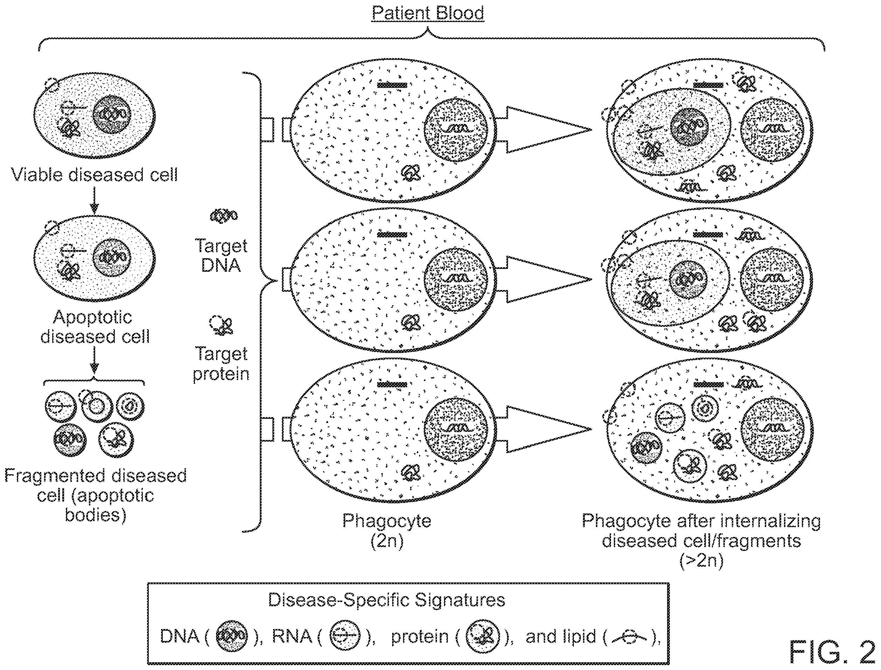

**FIG. 2** schematically depicts one proposed pathway leading to acquisition of a disease or condition-specific markers (e.g., DNA, RNA, protein and lipid markers) by phagocytic cells. Blood phagocytes engulf viable circulating diseased cells, apoptotic diseased cells, and/or fragmented diseased cells. Accordingly, the disease or condition-specific markers (e.g., DNAs, RNAs, proteins, or lipids) that are contained within these diseased cells/fragments are also internalized by phagocytic cells, which then become >2n phagocytic cells containing and/or expressing these specific markers. By contrast, phagocytic cells that do not internalize these diseased cells/fragments, and thus, do not contain or express these markers, and remain DNA content of 2n.

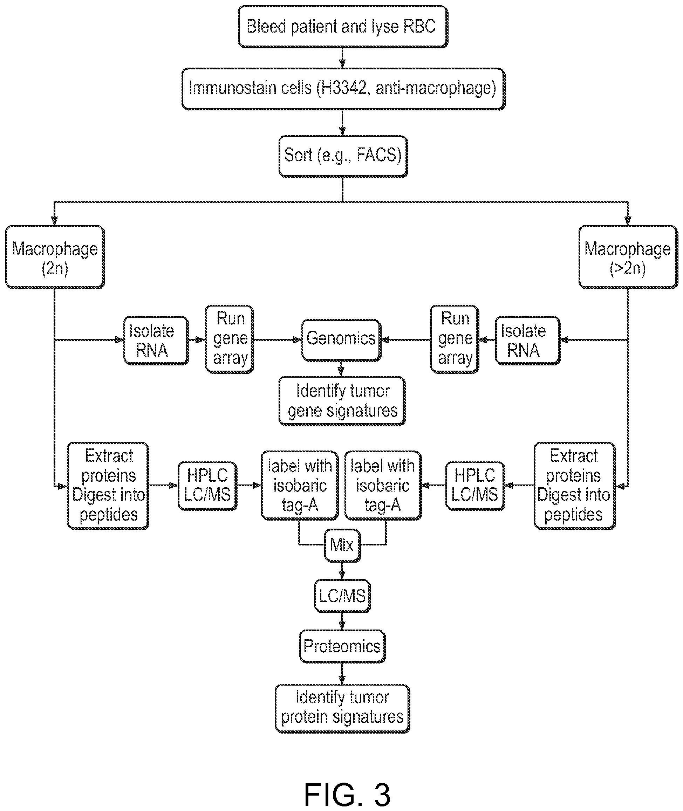

**FIG. 3** schematically depicts a general flowchart of one description of a method described herein.

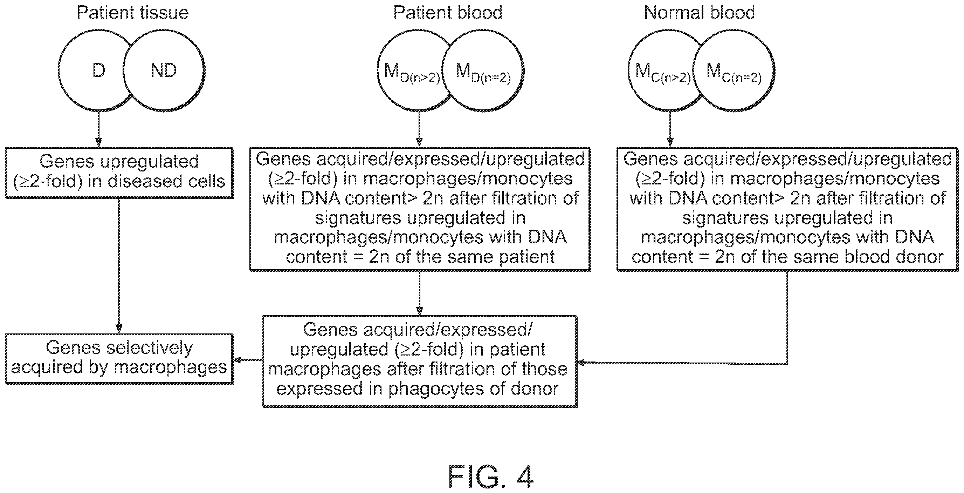

**FIG. 4** schematically depicts one description of a method described hereinfor identifying one or more markers of a disease or condition. D represents diseased tissues/cells from a patient having a disease or condition; and ND represents not-diseased tissues/cells from the patient; MD(N>2) represents macrophages having a DNA content of >2n taken from a patient with the disease or condition; MD(N=2) represents macrophages having a DNA content of =2n taken from the patient; MC(N>2) represents macrophages having a DNA content of >2n taken from a control subject not having the disease or condition; MC(N=2) represents macrophages having a DNA content of >2n taken from the control subject.

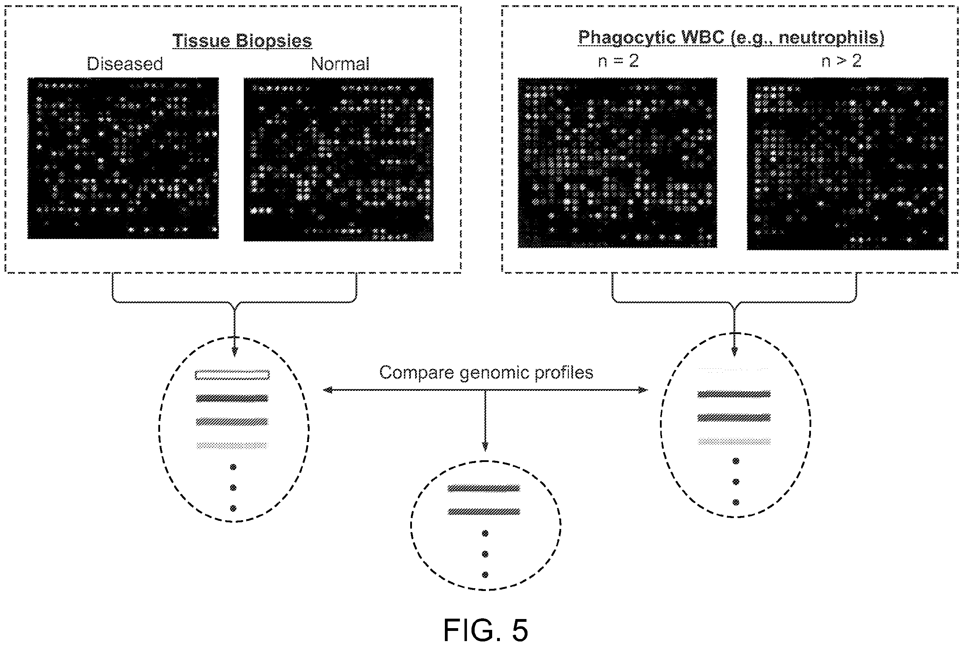

**FIG. 5** schematically depicts one description of a method described hereinfor identifying disease or condition-specific markers selectively acquired/expressed by >2n phagocytic cells of a patient.

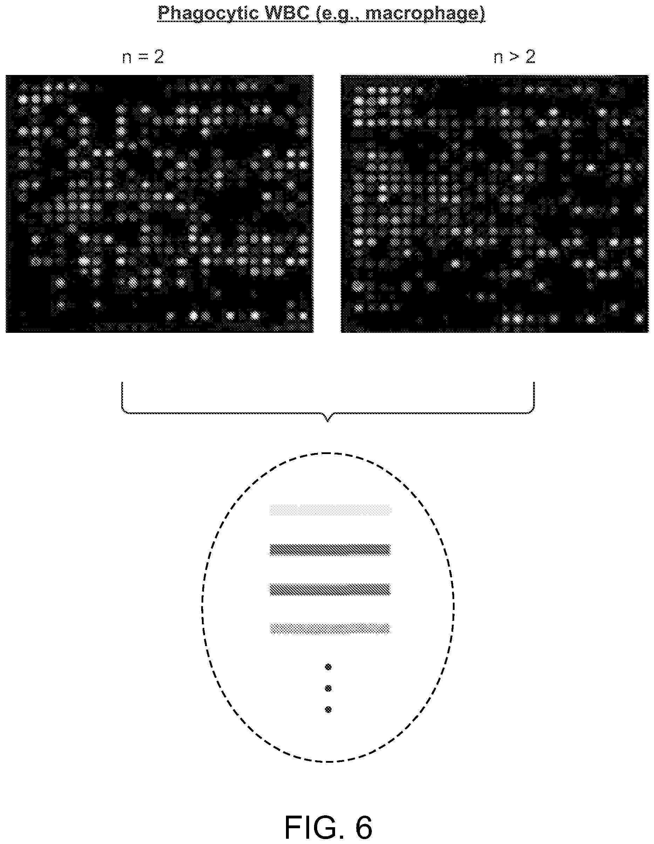

**FIG. 6** schematically depicts one description of a method described hereinfor diagnosing/detecting a disease or condition by comparing expression profiles obtained from arrays.

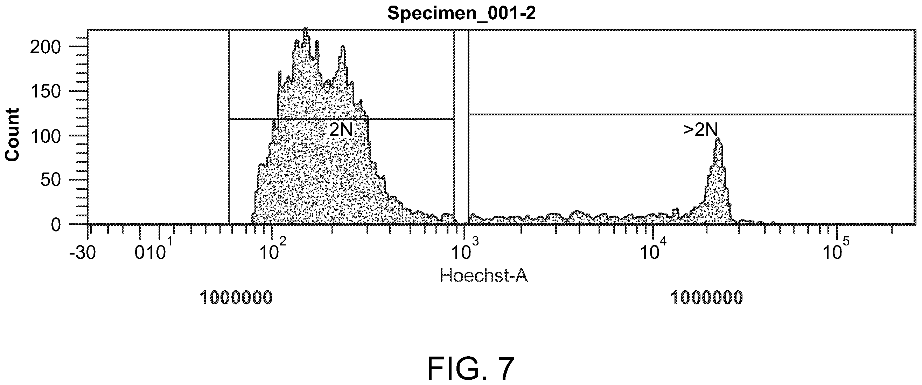

**FIG. 7** shows a fluorescence activated cell sorting (FACS) profile of human white blood cells previously stained with Hoechst 33342. The results demonstrate that white blood cells were separated into a population of >2n phagocytic cells and a population of =2n phagocytic cells. Each cell population has ∼10⁶ cells.

### Detailed Description

Unless otherwise defined herein, scientific and technical terms used in this application shall have the meanings that are commonly understood by those of ordinary skill in the art. Generally, nomenclature used in connection with, and techniques of, cell and tissue culture, molecular biology, cell and cancer biology, neurobiology, neurochemistry, virology, immunology, microbiology, pharmacology, genetics and protein and nucleic acid chemistry, described herein, are those well known and commonly used in the art.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

A "patient", "subject", or "individual" are used interchangeably and refer to either a human or a non-human animal. These terms include mammals, such as humans, primates, livestock animals (e.g., bovines, porcines), companion animals (e.g., canines, felines) and rodents (e.g., mice and rats).

As used herein, a control subject refers to any individual that has not been diagnosed as having the disease or condition being assayed. The terms "normal control", "healthy control", and "not-diseased cells" likewise mean a sample (e.g., cells, serum, tissue) taken from a source (e.g., subject, control subject, cell line) that does not have the condition or disease being assayed and therefore may be used to determine the baseline for the condition or disorder being measured. It is also understood that the control subject, normal control, and healthy control, include data obtained and used as a standard, i.e. it can be used over and over again for multiple different subjects. In other words, for example, when comparing a subject sample to a control sample, the data from the control sample could have been obtained in a different set of experiments, for example, it could be an average obtained from a number of healthy subjects and not actually obtained at the time the data for the subject was obtained.

The term "diagnosis" as used herein refers to methods by which the skilled artisan can estimate and/or determine whether or not a patient is suffering from a given disease or condition. The skilled artisan often makes a diagnosis on the basis of one or more diagnostic indicators, e.g., a marker, the presence, absence, amount, or change in amount of which is indicative of the presence, severity, or absence of the condition.

The term "prognosis" as used herein refers to is used herein to refer to the likelihood of a disease or condition progression, including recurrence of a disease or condition.

### Description of Methods

Described are methods for diagnosing or aiding in the diagnosis of a disease or condition by comparing profiles (e.g., gene/protein/lipid/carbohydrate expression profiles, genotypes, gene copy number, gene dosage, DNA methylation, etc.) of disease or condition-associated markers (e.g., nucleic acids, proteins, lipids, carbohydrates, metabolites) between phagocytic cells having different DNA contents (>2n vs. =2n) taken from the same individual.

Also described are methods for assessing the risk of developing a disease or condition, prognosing said disease, monitoring said disease progression or regression, assessing the efficacy of a treatment, or identifying a compound capable of ameliorating or treating said disease or condition.

Such a subject-specific profile comparison eliminates the dependence on a population-derived average profile for a particular disease or condition, which may introduce error into the detection or diagnosis of a particular disease or condition in the subject. The methods described allow detection, diagnosis, and treatment to be personalized to the individual.

The methods described (i) have high specificity, sensitivity, and accuracy and are capable of detecting disease or condition-specific markers present within a bodily fluid sample, cells or tissues; and (ii) eliminate the "inequality of baseline" that is known to occur among individuals due to intrinsic (e.g., age, gender, ethnic background, health status and the like) and temporal variations in marker expression. Accordingly, in certain descriptions, provided are noninvasive assays for the early detection of a disease or condition, i.e., before the disease can be diagnosed by conventional diagnostic techniques, e.g., imaging techniques, and, therefore, provide a foundation for improved decision-making relative to the needs and strategies for intervention, prevention, and treatment of individuals with such disease or condition.

The methods described can be used together with any known diagnostic methods, such as physical inspection, visual inspection, biopsy, scanning, histology, radiology, imaging, ultrasound, use of a commercial kit, genetic testing, immunological testing, analysis of bodily fluids, or monitoring neural activity.

Phagocytic cells that can be used in the methods described include all types of cells that are capable of ingesting various types of substances (e.g., apoptotic cells, infectious agents, dead cells, viable cells, cell-free DNAs, cell-free RNAs, cell-free proteins). In some descriptions, the phagocytic cells are professional phagocytic cells, such as neutrophils, macrophages, monocytes, dendritic cells, foam cells, mast cells, or eosinophils. In some descriptions, the phagocytic cells are non-professional phagocytic cells, such as epithelial cells, endothelial cells, fibroblasts, or mesenchymal cells. In other descriptions, the phagocytic cells can be a mixture of different types of phagocytic cells.

As used herein, "the >2n phagocytic cells" refer to phagocytic cells that have a DNA content of greater than 2n, while "the =2n phagocytic cells" refer to phagocytic cells that have a DNA content of 2n. Some phagocytic cells engulf live/dying/dead diseased cells (and subcellular fragments thereof) and/or cell-free disease-specific nucleic acids, proteins, carbohydrates and/or lipids present in bodily fluids. Such phagocytosis leads to the internalization of these disease markers into the phagocytic cell and, therefore, the DNA content of these phagocytic cells will become greater than 2n. By contrast, some phagocytic cells have not engulfed living/dying/dead diseased cells or fragments and/or cell-free disease-specific nucleic acids, proteins, lipids, and/or carbohydrates present in bodily fluids. The DNA contents of this group of phagocytic cells remain 2n. In some descriptions, the disease-specific markers (e.g., DNA with disease-specific mutations) can be expressed by the >2n phagocytic cells. For example, the mutated DNA of diseased cells is integrated into the normal DNA of the >2n phagocytic cells. The subsequent transcription of the "integrated" DNA of the >2n phagocytic cells into RNA and the translation of the latter into proteins produces a phenotype different from the phagocytic cells that have not phagocytosed the diseased cells (i.e., the =2n phagocytic cells). In other descriptions, the internalized disease-specific markers are not expressed by the >2n phagocytic cells. The markers may be translocated onto the membranes of the >2n phagocytic cells, or secreted out by the >2n phagocytic cells.

As used herein, a "profile" of a marker of a disease or condition can broadly refer to any information concerning the marker. This information can be either qualitative (e.g., presence or absence) or quantitative (e.g., levels, copy numbers, or dosages). In some descriptions, a profile of a marker can indicate the absence of this marker. The profile can be a nucleic acid (e.g., DNA or RNA) profile, a protein profile, a lipid profile, a carbohydrate profile, a metabolite profile, or a combination thereof. A "marker" as used herein generally refers to an analyte which is differentially detectable in phagocytes and is indicative of the presence of a disease or condition. An analyte is differentially detectable if it can be distinguished quantitatively or qualitatively in phagocytes.

The methods described can be applied to various diseases or conditions. Exemplar diseases or conditions are a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a mitochondrial disorder, a respiratory-gastrointestinal tract disease or condition, a reproductive disease or condition, an ophthalmic disease or condition, a musculo-skeletal disease or condition, or a dermal disease or condition.

As used herein, the term "cardiovascular disease or condition" refers to any condition that affects systems of heart or blood vessels (arteries and veins). Examples of cardiovascular diseases include, but are not limited to myocardial infarction, coronary artery disease, percutaneous transluminal coronary angioplasty (PTCA), coronary artery bypass surgery (CABG), restenosis, peripheral arterial disease, stroke, abdominal aorta aneurysm, intracranial aneurysm, large artery atherosclerotic stroke, cardiogenic stroke, an early onset myocardial infarction, heart failure, pulmonary embolism, acute coronary syndrome (ACS), angina, cardiac hypertrophy, arteriosclerosis, myocarditis, pancarditis, endocarditis, hypertension, congestive heart failure, atherosclerosis, cerebrovascular disease, declining cardiac health, ischemic heart disease, pericarditis, cardiogenic shock, alcoholic cardiomyopathy, congenital heart disease, ischemic cardiomyopathy, hypertensive cardiomyopathy, valvular cardiomyopathy, inflammatory cardiomyopathy, cardiomyopathy secondary to a systemic metabolic disease, dilated cardiomyopathy, hypertrophic cardiomyopathy, arrhythmogenic right ventricular cardiomyopathy, restrictive cardiomyopathy, noncompaction cardiomyopathy, valvular heart disease, hypertensive heart disease, myocardial ischemic attack, unstable angina, myocardial rupture, cardiogenic shock, embolism, deep vein thrombosis, arrhythmia, arrhythmogenic right ventricular cardiomyopathy, diabetic cardiomyopathy, mitral regurgitation, mitral valve prolapse, peripheral vascular disease, artery disease, carotid artery disease, deep vein thrombosis, venous diseases, cerebrovascular disease, arterial aneurysm, left ventricular hypertrophy, hypertensive renal disease, hypertensive retinal disease, vasculitis, left main disease, arterial vascular disease, venous vascular disease, thrombosis of the microcirculation, a transient cerebrovascular accident, limb ischemia, aneurysm, thrombosis, superficial venous thrombosis, and deep venous thrombosis.

As used herein, the term "kidney-associated disease or condition" refers to any disease or condition that affects kidney or renal system. Examples of kidney-associated disease include, but are not limited to, chronic kidney diseases, primary kidney diseases, non-diabetic kidney diseases, glomerulonephritis, interstitial nephritis, diabetic kidney diseases, diabetic nephropathy, glomerulosclerosis, rapid progressive glomerulonephritis, renal fibrosis, Alport syndrome, IDDM nephritis, mesangial proliferative glomerulonephritis, membrano proliferative glomerulonephritis, crescentic glomerulonephritis, renal insterstitial fibrosis, focal segmental glomerulosclerosis, membranous nephropathy, minimal change disease, pauci-immune rapid progressive glomerulonephritis, IgA nephropathy, polycystic kidney disease, Dent's disease, nephrocytinosis, Heymann nephritis, autosomal dominant (adult) polycystic kidney disease, autosomal recessive (childhood) polycystic kidney disease, acute kidney injury, nephrotic syndrome, renal ischemia, podocyte diseases or disorders, proteinuria, glomerular diseases, membranous glomerulonephritis, focal segmental glomerulonephritis, pre-eclampsia, eclampsia, kidney lesions, collagen vascular diseases, benign orthostatic (postural) proteinuria, IgM nephropathy, membranous nephropathy, sarcoidosis, diabetes mellitus, kidney damage due to drugs, Fabry's disease, aminoaciduria, Fanconi syndrome, hypertensive nephrosclerosis, interstitial nephritis, Sickle cell disease, hemoglobinuria, myoglobinuria, Wegener's Granulomatosis, Glycogen Storage Disease Type 1, chronic kidney disease, chronic renal failure, low Glomerular Filtration Rate (GFR), nephroangiosclerosis, lupus nephritis, ANCA-positive pauci-immune crescentic glomerulonephritis, chronic allograft nephropathy, nephrotoxicity, renal toxicity, kidney necrosis, kidney damage, glomerular and tubular injury, kidney dysfunction, nephritic syndrome, acute renal failure, chronic renal failure, proximal tubal dysfunction, acute kidney transplant rejection, chronic kidney transplant refection, non IgA mesangioproliferative glomerulonephritis, postinfectious glomerulonephritis, vasculitides with renal involvement of any kind, any hereditary renal disease, any interstitial nephritis, renal transplant failure, kidney cancer, kidney disease associated with other conditions (e.g., hypertension, diabetes, and autoimmune disease), Dent's disease, nephrocytinosis, Heymann nephritis, a primary kidney disease, a collapsing glomerulopathy, a dense deposit disease, a cryoglobulinemia- associated glomerulonephritis, an Henoch- Schonlein disease, a postinfectious glomerulonephritis, a bacterial endocarditis, a microscopic polyangitis, a Churg-Strauss syndrome, an anti-GBM-antibidy mediated glomerulonephritis, amyloidosis, a monoclonal immunoglobulin deposition disease, a fibrillary glomerulonephritis, an immunotactoid glomerulopathy, ischemic tubular injury, a medication-induced tubulo-interstitial nephritis, a toxic tubulo-interstitial nephritis, an infectious tubulo-interstitial nephritis, a bacterial pyelonephritis, a viral infectious tubulo-interstitial nephritis which results from a polyomavirus infection or an HIV infection, a metabolic-induced tubulo-interstitial disease, a mixed connective disease, a cast nephropathy, a crystal nephropathy which may results from urate or oxalate or drug-induced crystal deposition, an acute cellular tubulo- interstitial allograft rejection, a tumoral infiltrative disease which results from a lymphoma or a post-transplant lymphoproliferative disease, an obstructive disease of the kidney, vascular disease, a thrombotic microangiopathy, a nephroangiosclerosis, an atheroembolic disease, a mixed connective tissue disease, a polyarteritis nodosa, a calcineurin-inhibitor induced- vascular disease, an acute cellular vascular allograft rejection, an acute humoral allograft rejection, early renal function decline (ERFD), end stage renal disease (ESRD), renal vein thrombosis, acute tubular necrosis, acute interstitial nephritis, established chronic kidney disease, renal artery stenosis, ischemic nephropathy, uremia, drug and toxin-induced chronic tubulointerstitial nephritis, reflux nephropathy, kidney stones, Goodpasture's syndrome, and hydronephrosis.

As used herein, the term "prenatal or pregnancy-related disease or condition" refers to any disease, disorder, or condition affecting a pregnant woman, embryo, or fetus. Prenatal or pregancy-related conditions can also refer to any disease, disorder, or condition that is associated with or arises, either directly or indirectly, as a result of pregnancy. These diseases or conditions can include any and all birth defects, congenital conditions, or hereditary diseases or conditions. Examples of prenatal or pregnancy-related diseases include, but are not limited to, Rhesus disease, hemolytic disease of the newborn, beta-thalassemia, sex determination, determination of pregnancy, a hereditary Mendelian genetic disorder, chromosomal aberrations, a fetal chromosomal aneuploidy, fetal chromosomal trisomy, fetal chromosomal monosomy, trisomy 8, trisomy 13 (Patau Syndrom), trisomy 16, trisomy 18 (Edwards syndrome), trisomy 21 (Down syndrome), X-chromosome linked disorders, trisomy X (XXX syndrome), monosomy X (Turner syndrome), XXY syndrome, XYY syndrome, XYY syndrome, XXXY syndrome, XXYY syndrome, XYYY syndrome, XXXXX syndrome, XXXXY syndrome, XXXYY syndrome, XXYYY syndrome, Fragile X Syndrome, fetal growth restriction, cystic fibrosis, a hemoglobinopathy, fetal death, fetal alcohol syndrome, sickle cell anemia, hemophilia, Klinefelter syndrome, dup(17)(p11.2p1.2) syndrome, endometriosis, Pelizaeus-Merzbacher disease, dup(22)(q11.2q11.2) syndrome, cat eye syndrome, cri-du-chat syndrome, Wolf-Hirschhorn syndrome, Williams -Beuren syndrome, Charcot-Marie-Tooth disease, neuropathy with liability to pressure palsies, Smith-Magenis syndrome, neurofibromatosis, Alagille syndrome, Velocardiofacial syndrome, DiGeorge syndrome, steroid sulfatase deficiency, Prader-Willi syndrome, Kallmann syndrome, microphthalmia with linear skin defects, adrenal hypoplasia, glycerol kinase deficiency, Pelizaeus-Merzbacher disease, testis-determining factor on Y, azospermia (factor a), azospermia (factor b), azospermia (factor c), lp36 deletion, phenylketonuria, Tay-Sachs disease, adrenal hyperplasia, Fanconi anemia, spinal muscular atrophy, Duchenne's muscular dystrophy, Huntington's disease, myotonic dystrophy, Robertsonian translocation, Angelman syndrome, tuberous sclerosis, ataxia telangieltasia, open spina bifida, neural tube defects, ventral wall defects, small-for-gestational-age, congenital cytomegalovirus, achondroplasia, Marfan's syndrome, congenital hypothyroidism, congenital toxoplasmosis, biotinidase deficiency, galactosemia, maple syrup urine disease, homocystinuria, medium- chain acyl Co-A dehydrogenase deficiency, structural birth defects, heart defects, abnormal limbs, club foot, anencephaly, arhinencephaly/holoprosencephaly, hydrocephaly, anophthalmos/microphthalmos, anotia/microtia, transposition of great vessels, tetralogy of Fallot, hypoplastic left heart syndrome, coarctation of aorta, cleft palate without cleft lip, cleft lip with or without cleft palate, oesophageal atresia/stenosis with or without fistula, small intestine atresia/stenosis, anorectal atresia/stenosis, hypospadias, indeterminate sex, renal agenesis, cystic kidney, preaxial Polydactyly, limb reduction defects, diaphragmatic hernia, blindness, cataracts, visual problems, hearing loss, deafness, X-linked adrenoleukodystrophy, Rett syndrome, lysosomal disorders, cerebral palsy, autism, aglossia, albinism, ocular albinism, oculocutaneous albinism, gestational diabetes, Arnold-Chiari malformation, CHARGE syndrome, congenital diaphragmatic hernia, brachydactlia, aniridia, cleft foot and hand, heterochromia, Dwarnian ear, Ehlers Danlos syndrome, epidermolysis bullosa, Gorham's disease, Hashimoto's syndrome, hydrops fetalis, hypotonia, Klippel-Feil syndrome, muscular dystrophy, osteogenesis imperfecta, progeria, Smith Lemli Opitz symdrom, chromatelopsia, X-linked lymphoproliferative disease, omphalocele, gastroschisis, pre-eclampsia, eclampsia, pre-term labor, premature birth, miscarriage, delayed intrauterine growth, ectopic pregnancy, hyperemesis gravidarum, morning sickness, or likelihood for successful induction of labor.

As used herein, the term "a neurological or neuropsychiatric disease or condition" refers to any disease or condition that affects nervous systems. Examples of neurological or neuropsychiatry diseases or conditions include, but are not limited to, head trauma, stroke, stroke, ischemic stroke, hemorrhagic stroke, subarachnoid hemorrhage, intra cranial hemorrhage, transient ischemic attack, vascular dementia, corticobasal ganglionic degeneration, encephalitis, epilepsy, Landau-Kleffner syndrome, hydrocephalus, pseudotumor cerebri, thalamic diseases, meningitis, myelitis, movement disorders, essential tremor, spinal cord diseases, syringomyelia, Alzheimer's disease (early onset), Alzheimer's disease (late onset), multi-infarct dementia, Pick's disease, Huntingdon's disease, Parkinson's disease, Parkinson syndromes, dementia, frontotemporal dementia, corticobasal degeneration, multiple system atrophy, progressive supranuclear palsy, Lewy body disease, Creutzfeldt- Jakob disease, Dandy- Walker syndrome, Friedreich ataxia, Machado-Joseph disease, migraine, schizophrenia, mood disorders and depression, dementia with lewy bodies (DLB), frontotemporal dementia (FTD), various forms of vascular dementia (VD), subcortical vascular dementia (Binswanger's disease), autism, developmental retardations, motor neuron diseases, amyotrophic lateral sclerosis (ALS), neuronal or brain damage, hypoxia of the brain, cerebral palsy (CP), memory disorders, movement disorders, corticalbasal ganglionic degeneration, forms of multiple system atrophy, stroke- related disorders, cerebrovascular accidents, post-irradiation encephalopathy with seizures, vascular Parkinsonism, thalamic cerebrovascular accidents, chronic inflammatory demyelinating polyneuropathy, alcohol related dementia, semantic dementia, ataxia, atypical Parkinsonism, dystonia, progressive supranuclear palsy, essential tremor, mild cognitive impairment, amyotrophic lateral sclerosis, multiple sclerosis, neuropathies, Pick's disease, congophilic amyloid angiopathy, Creutzfeldt- Jakob Disease, AIDS dementia complex, depression, anxiety disorder, phobia, Bell's Palsy, epilepsy, encephalitis, neuromuscular disorders, neurooncological disorders, brain tumors, neurovascular disorders, neuroimmunological disorders, neurootological disease, neurotrauma including spinal cord injury, pain including neuropathic pain, pediatric neurological and neuropsychiatric disorders, sleep disorders, Tourette syndrome, corticalbasal ganglionic degeneration, alcohol related dementia, semantic dementia, Alzheimer's disease combined with multi-infarct dementia, Alzheimer's disease combined with Lewy body dementia, Parkinson's disease combined with Lewy body dementia, Alzheimer's and Parkinson's disease combined with Lewy body dementia, frontotemporal dementia combined with chronic inflammatory demyelinating polyneuropathy, attention deficit hyperactivity disorder, schizophrenia, obsessive- compulsive disorder, mental retardation, autistic spectrum disorders, opsoclonus- myoclonus syndrome (OMS) seizures, articulation disorder, learning disabilities (i.e., reading or arithmetic), verbal or performance aptitude deficits, attention deficit disorder, amyloid diseases, prion diseases, Tauopathies, Alpha-Synucleinopathies, addictive states such as those caused by at least one of: cocaine, nicotine, alcohol, food, ecstasy, kat, caffeine, opium, heroin, marijuana, amphetamine, methamphetamine or gambling, and Fabry's disease.

As used herein, the term "an autoimmune or immune -related disease or condition" refers to any disease or condition that affects the function of immune systems. Examples of autoimmune or immune-related diseases or conditions include, but are not limited to, antiphospholipid syndrome, systemic lupus erythematosus, rheumatoid arthritis, autoimmune vasculitis, celiac disease, autoimmune thyroiditis, post-transfusion immunization, maternal-fetal incompatibility, transfusion reactions, immunological deficiency such IgA deficiency, common variable immunodeficiency, drug-induced lupus, diabetes mellitus, Type I diabetes, Type II diabetes, juvenile onset diabetes, juvenile rheumatoid arthritis, psoriatic arthritis, multiple sclerosis, immunodeficiency, allergies, asthma, psoriasis, atopic dermatitis, allergic contact dermatitis, chronic skin diseases, amyotrophic lateral sclerosis, chemotherapy-induced injury, graft- vs-host diseases, bone marrow transplant rejection, Ankylosing spondylitis, atopic eczema, Pemphigus, Behcet's disease, chronic fatigue syndrome fibromyalgia, chemotherapy-induced injury, myasthenia gravis, glomerulonephritis, allergic retinitis, systemic sclerosis, subacute cutaneous lupus erythematosus, cutaneous lupus erythematosus including chilblain lupus erythematosus, Sjogren's syndrome, autoimmune nephritis, autoimmune vasculitis, autoimmune hepatitis, autoimmune carditis, autoimmune encephalitis, autoimmune mediated hematological diseases, lc-SSc (limited cutaneous form of scleroderma), dc-SSc (diffused cutaneous form of scleroderma), autoimmune thyroiditis (AT), Grave's disease (GD), myasthenia gravis, multiple sclerosis (MS), ankylosing spondylitis, transplant rejection, immune aging, rheumatic/autoimmune diseases, mixed connective tissue disease, spondyloarthropathy, psoriasis, psoriatic arthritis, myositis, scleroderma, dermatomyositis, autoimmune vasculitis, mixed connective tissue disease, idiopathic thrombocytopenic purpura, Crohn's disease, human adjuvant disease, osteoarthritis, juvenile chronic arthritis, a spondyloarthropathy, an idiopathic inflammatory myopathy, systemic vasculitis, sarcoidosis, autoimmune hemolytic anemia, autoimmune thrombocytopenia, thyroiditis, immune-mediated renal disease, a demyelinating disease of the central or peripheral nervous system, idiopathic demyelinating polyneuropathy, Guillain-Barre syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease, psoriatic arthritis, psoriasis, dermatitis, polymyositis/dermatomyositis, toxic epidermal necrolysis, systemic scleroderma and sclerosis, responses associated with inflammatory bowel disease, Crohn's disease, ulcerative colitis, respiratory distress syndrome, adult respiratory distress syndrome (ARDS), meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, mysathenia gravis, antigen-antibody complex mediated diseases, anti-glomerular basement membrane disease, anti- phospholipid antibody syndrome, allergic neuritis, Bechet disease, Castleman's syndrome, Goodpasture's syndrome, Lambert-Eaton Myasthenic Syndrome, Reynaud's syndrome, Sjorgen's syndrome, Stevens- Johnson syndrome, pemphigoid bullous, pemphigus, autoimmune polyendocrinopathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimmune orchitis and oophoritis, primary hypothyroidism, autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Sheehan's syndrome, autoimmune hepatitis, lymphoid interstitial pneumonitis (HIV), bronchiolitis obliterans (non- transplant) vs NSIP, Guillain-Barre' Syndrome, large vessel vasculitis (including polymyalgia rheumatica and giant cell (Takayasu's) arteritis), medium vessel vasculitis (including Kawasaki's disease and polyarteritis nodosa), ankylosing spondylitis, Berger's disease (IgA nephropathy), rapidly progressive glomerulonephritis, primary biliary cirrhosis, Celiac sprue (gluten enteropathy), cryoglobulinemia, and amyotrophic lateral sclerosis (ALS).

As used herein, the term "cancer" refers to various types of malignant neoplasms, most of which can invade surrounding tissues, and may metastasize to different sites (see, for example, PDR Medical Dictionary, 1st edition (1995)). The terms "neoplasm" and "tumor" refer to an abnormal tissue that grows by cellular proliferation more rapidly than normal and continues to grow after the stimuli that initiated proliferation is removed. Id. Such abnormal tissue shows partial or complete lack of structural organization and functional coordination with the normal tissue which may be either benign (i.e., benign tumor) or malignant (i.e., malignant tumor). Examples of general categories of cancer include, but are not limited to, carcinomas (i.e., malignant tumors derived from epithelial cells such as, for example, common forms of breast, prostate, lung and colon cancer), sarcomas (i.e., malignant tumors derived from connective tissue or mesenchymal cells), lymphomas (i.e., malignancies derived from hematopoietic cells), leukemias (i.e., malignancies derived from hematopoietic cells), germ cell tumors (i.e., tumors derived from totipotent cells. In adults most often found in the testicle or ovary; in fetuses, babies and young children, most often found on the body midline, particularly at the tip of the tailbone), blastic tumors (i.e., a typically malignant tumor which resembles an immature or embryonic tissue) and the like. Examples of the types of neoplasms intended to be encompassed by the present descriptioninclude but are not limited to those neoplasms associated with cancers of neural tissue, blood forming tissue, breast, skin, bone, prostate, ovaries, uterus, cervix, liver, lung, brain, larynx, gallbladder, pancreas, rectum, parathyroid, thyroid, adrenal gland, immune system, head and neck, colon, stomach, bronchi, and/or kidneys.

As used herein, "treating" a disease or condition refers to taking steps to obtain beneficial or desired results, including clinical results. Beneficial or desired clinical results include, but are not limited to, alleviation or amelioration of one or more symptoms associated with diseases or conditions.

As used herein, "administering" or "administration of a compound or an agent to a subject can be carried out using one of a variety of methods known to those skilled in the art. For example, a compound or an agent can be administered, intravenously, arterially, intradermally, intramuscularly, intraperitonealy, intravenously, subcutaneous ly, ocularly, sublingually, orally (by ingestion), intranasally (by inhalation), intraspinally, intracerebrally, and transdermally (by absorbtion, e.g., through a skin duct). A compound or agent can also appropriately be introduced by rechargeable or biodegradable polymeric devices or other devices, e.g., patches and pumps, or formulations, which provide for the extended, slow, or controlled release of the compound or agent. Administering can also be performed, for example, once, a plurality of times, and/or over one or more extended periods. In some descriptions, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient. In some descriptions, a compound or an agent is administered orally, e.g., to a subject by ingestion, or intravenously, e.g., to a subject by injection. In some descriptions, the orally administered compound or agent is in an extended release or slow release formulation, or administered using a device for such slow or extended release.

In certain descriptions, markers used in the methods described are up-regulated or activated in the >2n phagocytic cells compared to the =2n phagocytic cells. In certain descriptions, markers used in the methods described are down-regulated or inhibited in the >2n phagocytic cells compared to the =2n phagocytic cells. Different diseases or conditions can be associated with either up-regulation (or activation) or down-regulation (or inhibition) of different markers. As used herein, "up-regulation or up-regulated" can refer to an increase in expression levels (e.g., gene expression or protein expression), gene copy numbers, gene dosages, and other qualitative or quantitative detectable state of the markers. Similarly, "down-regulation or down-regulated" can refer to an increase in expression levels, gene copy numbers, gene dosages, and other qualitative or quantitative detectable state of the markers. As used herein, "activation or activated" can refer to an active state of the marker, e.g., a phosphorylation state, a DNA methylation state, or a DNA acetylation state. Similarly, "inhibition or inhibited" can refer to a repressed state or an inactivated state of the marker, e.g., a de-phosphorylation state, a ubiquitination state, a DNA de-methylation state.

In certain descriptions, methods described also comprise at least one of the following steps before determination of various profiles: i) lysing the >2n phagocytic cells and the =2n phagocytic cells; and ii) extracting cellular contents from the lysed >2n phagocytic cells and the lysed =2n phagocytic cells. In certain descriptions, the cellular contents of the >2n phagocytic cells comprise various types of materials that they have engulfed, such as, viable diseased cells, dead diseased cells, apoptotic diseased cells, circulating tumor cells, infectious agents, fetal cells, trophoblasts, or fragments thereof. In certain descriptions, at least one or more markers of a disease or condition are present in the cellular contents of the >2n phagocytic cells. In certain descriptions, there is no marker present in the cellular contents of the =2n phagocytic cells.

In certain descriptions, methods described further comprise comparing the identified difference of the disease or condition-specific markers to a repository of at least one markers known in the art. Such comparison can further confirm the presence of the disease or condition. In some descriptions, the repository of the known markers can be obtained by data mining. The term "data mining", as used herein, refers to a process of finding new data patterns, relations, or correlations derived from the known data of the databases and of extracting practicable information in the future. Typically a computer-based system can be trained on data to perform the data mining, e.g., to classify the input data and then subsequently used with new input data to make decisions based on the training data. These systems include, but are not limited, expert systems, fuzzy logic, nonlinear regression analysis, multivariate analysis, decision tree classifiers, and Bayesian belief networks.

In certain descriptions, the >2n phagocytic cells and the =2n phagocytic cells are isolated from a bodily fluid sample, tissues, or cells. Exemplar bodily fluid sample can be whole blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, or ocular fluid. In some descriptions, the >2n phagocytic cells and the =2n phagocytic cells are isolated from white blood cells. In certain descriptions, the >2n phagocytic cells and the =2n phagocytic cells are separated from a population of phagocytic cells.

In the methods described, cell separation/isolation/purification methods are used to isolate populations of cells from bodily fluid sample, cells, or tissues of a subject. A skilled worker can use any known cell separation/isolation/purification techniques to isolate >2n phagocytic cells and =2n phagocytic cells from a bodily fluid, or to separate >2n phagocytic cells from =2n phagocytic cells. Exemplar techniques include, but are not limited to, using antibodies, flow cytometry, fluorescence activated cell sorting, filtration, gradient- based centrifugation, elution, microfluidics, magnetic separation technique, fluorescent-magnetic separation technique, nanostructure, quantum dots, high throughput microscope-based platform, or a combination thereof.

In certain descriptions, the >2n phagocytic cells and the =2n phagocytic cells are isolated by using a product secreted by the >2n phagocytic cells. In certain descriptions, the >2n phagocytic cells and the =2n phagocytic cells are isolated by using a cell surface target (e.g., receptor protein) on the surface of phagocytic cells. In some descriptions, the cell surface target is a protein that has been engulfed by the >2n phagocytic cells. In some descriptions, the cell surface target is expressed by the >2n phagocytic cells on their plasma membranes. In some descriptions, the cell surface target is an exogenous protein that is translocated on the plasma membranes, but not expressed by the >2n phagocytic cells. In some descriptions, the cell surface target is a marker of the disease or condition to be detected.

In certain methods described herein, analytes include nucleic acids, proteins, lipids, carbohydrates, metabolites, or any combinations of these. In certain descriptions of the methods described herein, markers include nucleic acids, proteins, lipids, carbohydrates, metabolites, or any combinations of these. As used herein, the term "nucleic acid" is intended to include DNA molecules (e.g., cDNA or genomic DNA), RNA molecules (e.g., mRNA), DNA-RNA hybrids, and analogs of the DNA or RNA generated using nucleotide analogs. The nucleic acid molecule can be a nucleotide, oligonucleotide, double-stranded DNA, single- stranded DNA, multi-stranded DNA, complementary DNA, genomic DNA, non- coding DNA, messenger RNA (mRNAs), microRNA (miRNAs), small nucleolar RNA (snoRNAs), ribosomal RNA (rRNA), transfer RNA (tRNA), small interfering RNA (siRNA), heterogeneous nuclear RNAs (hnRNA), or small hairpin RNA (shRNA).

As used herein, the term "amino acid" includes organic compounds containing both a basic amino group and an acidic carboxyl group. Included within this term are natural amino acids (e.g., L-amino acids), modified and unusual amino acids (e.g., D-amino acids and β-amino acids), as well as amino acids which are known to occur biologically in free or combined form but usually do not occur in proteins. Natural protein occurring amino acids include alanine, arginine, asparagine, aspartic acid, cysteine, glutamic acid, glutamine, glycine, histidine, isoleucine, leucine, lysine, methionine, phenylalanine, serine, threonine, tyrosine, tryptophan, proline, and valine. Natural non-protein amino acids include arginosuccinic acid, citrulline, cysteine sulfuric acid, 3,4-dihydroxyphenylalanine, homocysteine, homoserine, ornithine, 3-monoiodotyrosine, 3,5-diiodotryosine, 3, 5, 5 -triiodothyronine, and 3,3',5,5'-tetraiodothyronine. Modified or unusual amino acids include D-amino acids, hydroxy lysine, 4-hydroxyproline, N-Cbz-protected amino acids, 2,4-diaminobutyric acid, homoarginine, norleucine, N- methylaminobutyric acid, naphthylalanine, phenylglycine, .alpha.-phenylproline, tert-leucine, 4-aminocyclohexylalanine, N-methyl-norleucine, 3 ,4-dehydroproline, N,N-dimethylaminoglycine, N-methylaminoglycine, 4-aminopiperidine-4- carboxylic acid, 6-aminocaproic acid, trans-4-(aminomethyl)- cyclohexanecarboxylic acid, 2-, 3-, and 4-(aminomethyl)-benzoic acid, 1- aminocyclopentanecarboxylic acid, 1-aminocyclopropanecarboxylic acid, and 2- benzyl-5-aminopentanoic acid.

As used herein, the term "peptide" includes compounds that consist of two or more amino acids that are linked by means of a peptide bond. Peptides may have a molecular weight of less than 10,000 Daltons, less than 5,000 Daltons, or less than 2,500 Daltons. The term "peptide" also includes compounds containing both peptide and non-peptide components, such as pseudopeptide or peptidomimetic residues or other non-amino acid components. Such compounds containing both peptide and non-peptide components may also be referred to as a "peptide analog."

As used herein, the term "protein" includes compounds that consist of amino acids arranged in a linear chain and joined together by peptide bonds between the carboxyl and amino groups of adjacent amino acid residues. Proteins used in methods described include, but are not limited to, amino acids, peptides, antibodies, antibody fragments, cytokines, lipoproteins, or glycoproteins.

As used herein, the term "antibody" includes polyclonal antibodies, monoclonal antibodies (including full length antibodies which have an immunoglobulin Fc region), antibody compositions with polyepitopic specificity, multispecific antibodies (e.g., bispecific antibodies, diabodies, and single-chain molecules, and antibody fragments (e.g., Fab or F(ab')2, and Fv). For the structure and properties of the different classes of antibodies, see e.g., Basic and Clinical Immunology, 8th Edition, Daniel P. Sties, Abba I. Terr and Tristram G. Parsolw (eds), Appleton & Lange, Norwalk, Conn., 1994, page 71 and Chapter 6.

As used herein, the term "cytokine" refers to a secreted protein or active fragment or mutant thereof that modulates the activity of cells of the immune system. Examples of cytokines include, without limitation, interleukins, interferons, chemokines, tumor necrosis factors, colony-stimulating factors for immune cell precursors, and the like.

As used herein, the term "lipoprotein" includes negatively charged compositions that comprise a core of hydrophobic cholesteryl esters and triglyceride surrounded by a surface layer of amphipathic phospholipids with which free cholesterol and apolipoproteins are associated. Lipoproteins may be characterized by their density (e.g. very-low-density lipoprotein (VLDL), low- density lipoprotein (LDL) and high density lipoprotein (HDL)), which is determined by their size, the relative amounts of lipid and protein. Lipoproteins may also be characterized by the presence or absence of particular modifications (e.g. oxidization, acetylation, or glycation).

As used herein, the term "glycoprotein" includes glycosides which have one or more oligo- or polysaccharides covalently attached to a peptide or protein. Exemplary glycoproteins can include, without limitation, immunoglobulins, members of the major histocompatibility complex, collagens, mucins, glycoprotein Ilb/IIIa, glycoprotein-41 (gp41) and glycoprotein- 120 (gpl2), follicle-stimulating hormone, alpha- fetoprotein, erythropoietin, transferrins, alkaline phosphatase, and lectins.

As used herein, the term "lipid" includes synthetic or naturally-occurring compounds which are generally amphipathic and biocompatible. Lipids typically comprise a hydrophilic component and a hydrophobic component. Exemplary lipids include, but are not limited to fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, sulfatide, free fatty acids, prostaglandins, triacylglycerol, diacylglycerol, monoacylglycerol, acyl-CoA, acylcarnitine, oxysterol, ceramide, cardiolipin, sphingoid base-1 -phosphate, shingosine, lyso-sphingomyelin,, gangliosides, plasmalogen, sulfatide, ceramide, low density lipoproteins (LDLs), very low density lipoproteins (VLDLs), high density lipoproteins (HDLs), sphingoid base-1- phosphates or derivatives thereof.

As used herein, the term "carbohydrate" includes, but is not limited to, compounds that contain oxygen, hydrogen and carbon atoms, typically (CH20)n wherein n is an integer. Exemplary carbohydrates include, but are not limited to, monosaccharides, disaccharides, polysaccharides, or oligosaccharides.

As used herein, the term "metabolite" includes any molecule used in metabolism. Metabolites can be products, substrates, or intermediates in metabolic processes. Included within this term are primary metabolites, secondary metabolites, organic metabolites, or inorganic metabolites. Metabolites include, without limitation, amino acids, peptides, acylcarnitines, monosaccharides, lipids and phospholipids, prostaglandins, hydroxyeicosatetraenoic acids, hydroxyoctadecadienoic acids, steroids, bile acids, and glycolipids and phospholipids. Exemplary metabolites can be sphingolipids, glycosphingolipids, sphingosine, ceramide, sphingomyelin, sphingosylphosphorylcholin, dihydrosphingosine, phoshatidylcholine, phosphatidylinositol, phosphatidylserine, lysophoshatidylcholine, lysophosphatidylinositol, lysophosphatidylserine, plasmenylphoshatidylcholine, plasmanylphoshatidylcholine, proteinogenic amino acids, Alanine, Aspartic acid, Glutamic acid, Phenylalanine, Glycine, Histidine, Leucine, Isoleucine, Lysine, Methionine, Proline, Arginine, Serine, Threonine, Valine, Tryptophan, Tyrosine, asymmetrical dimethyl arginine, symmetrical dimethyl arginine, Glutamine, Asparagine, Nitrotyrosine, Hydroxyproline, Kynurenine, 3 - Hydroxy kynurenine, non-proteinogenic amino acids, Ornithine, Citrulline, acylcarnitines, carnitine, free carnitine, acylcarnitine, hydroxylacylcarnitine, dicarboxylacylcarnitines, reducing monosaccharides, hexose, pentose, deoxyhexose, creatinine, creatine, spermidine spermine, putrescine, dopamine, serotonin, prostaglandins, hydoxyeicosatetraeneoic acid, Hydroxyoctadecadienoic acid, leukatrienes, thromboxanes, bile acids, sterols, cholesterols, vitamins and cofactors, drugs, and drug metabolites.

In some descriptions, profiles of at least one or more markers of a disease or condition are compared. This comparison can be quantitative or qualitative. Quantitative measurements can be taken using any of the assays described herein. For example, sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single- base extension sequencing, solid-phase sequencing, high-throughput sequencing, massively parallel signature sequencing, emulsion PC , sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.

Quantitative comparisons can include statistical analyses such as t-test, ANOVA, Krustal-Wallis, Wilcoxon, Mann- Whitney, and odds ratio. Quantitative differences can include differences in the levels of markers between profiles or differences in the numbers of markers present between profiles, and combinations thereof. Examples of levels of the markers can be, without limitation, gene expression levels, nucleic acid levels, protein levels, lipid levels, and the like. Qualitative differences can include, but are not limited to, activation and inactivation, protein degradation, nucleic acid degradation, and covalent modifications.

In certain descriptions, the profile is a nucleic acid profile, a protein profile, a lipid profile, a carbohydrate profile, a metabolite profile, or a combination thereof. The profile can be qualitatively or quantitatively determined.

A nucleic acid profile can be, without limitation, a genotypic profile, a single nucleotide polymorphism profile, a gene mutation profile, a gene copy number profile, a DNA methylation profile, a DNA acetylation profile, a chromosome dosage profile, a gene expression profile, or a combination thereof.

The nucleic acid profile can be determined by any methods known in the art to detect genotypes, single nucleotide polymorphisms, gene mutations, gene copy numbers, DNA methylation states, DNA acetylation states, chromosome dosages. Exemplar methods include, but are not limited to, polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole- time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, Hpall tiny fragment Enrichment by Ligation-mediated PCR (HELP) assay, radioactive acetate labeling assays, colorimetric DNA acetylation assay, chromatin immunoprecipitation combined with microarray (ChIP -on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, methyl-binding PCR analysis, or a combination thereof.

As used herein, the term "sequencing" is used in a broad sense and refers to any technique known in the art that allows the order of at least some consecutive nucleotides in at least part of a nucleic acid to be identified, including without limitation at least part of an extension product or a vector insert. Exemplar sequencing techniques include direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid- phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by- synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof. In some descriptions, sequencing comprises an detecting the sequencing product using an instrument, for example but not limited to an ABI PRISM® 377 DNA Sequencer, an ABI PRISM® 310, 3100, 3100-Avant, 3730, or 3730x1 Genetic Analyzer, an ABI PRISM® 3700 DNA Analyzer, or an Applied Biosystems SOLiD™ System (all from Applied Biosystems), a Genome Sequencer 20 System (Roche Applied Science), or a mass spectrometer. In certain descriptions, sequencing comprises emulsion PCR. In certain descriptions, sequencing comprises a high throughput sequencing technique, for example but not limited to, massively parallel signature sequencing (MPSS).

In further descriptions, a protein profile can be a protein expression profile, a protein activation profile, or a combination thereof. In some descriptions, a protein activation profile can comprise determining a phosphorylation state, an ubiquitination state, a myristoylation state, or a conformational state of the protein.

A protein profile can be detected by any methods known in the art for detecting protein expression levels, protein phosphorylation state, protein ubiquitination state, protein myristoylation state, or protein conformational state. In some descriptions, a protein profile can be determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole- time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof.

In some descriptions, a lipid profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole- time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof. Further methods for analyzing lipid content in a biological sample are known in the art (See, e.g., Kang et al. (1992) Biochim. Biophys. Acta. 1 128:267; Weylandt et al. (1996) Lipids 31 :977; J. Schiller et al. (1999) Anal. Biochem. 267:46; Kang et al. (2001) Proc. Natl. Acad. Sci. USA 98:4050; Schiller et al. (2004) Prog. Lipid Res. 43 :499). One exemplary method of lipid analysis is to extract lipids from a biological sample (e.g. using chloroform-methanol (2: 1, vol/vol) containing 0.005% butylated hydroxytoluene (BHT, as an antioxidant)), prepare fatty acid methyl esters (e.g., using 14% BF3 -methanol reagent), and quantify the fatty acid methyl esters (e.g., by HPLC, TLC, by gas chromatography-mass spectroscopy using commercially available gas chromatographs, mass spectrometers, and/or combination gas chromatograph/mass spectrometers). Fatty acid mass is determined by comparing areas of various analyzed fatty acids to that of a fixed concentration of internal standard.

In some descriptions, a carbohydrate profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole- time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

In some descriptions, a metabolite profile can be determind by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser desorption/ionization-time of flight (SELDI-TOF) mass spectrometry, quadrupole- time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

As used herein, the "difference" between different profiles detected by the methods described can refer to different gene copy numbers, different DNA, RNA, protein, lipid, or carbohydrate expression levels, different DNA methylation states, different DNA acetylation states, and different protein modification states. The difference can be a difference greater than 1 fold. In some descriptions, the difference is a 1.05-fold, 1.1-fold, 1.2-fold, 1.3-fold, 1.4-fold, 1.5-fold, 2-fold, 2.5- fold, 3-fold, 4-fold, 5-fold, 6-fold, 7-fold, 8-fold, 9-fold, or 10-fold difference. In some descriptions, the difference is any fold difference between 1-10, 2-10, 5-10, 10-20, or 10-100 folds.

A general principle of assays to detect markers involves preparing a sample or reaction mixture that may contain the marker (e.g., one or more of DNA, RNA, protein, polypeptide, carbohydrate, lipid, metabolite, and the like) and a probe under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one description of such a method, a sample from a subject, which is to be assayed for presence and/or concentration of marker, can be anchored onto a carrier or solid phase support. In another description, the reverse situation is possible, in which the probe can be anchored to a solid phase and a sample from a subject can be allowed to react as an unanchored component of the assay.

There are many established methods for anchoring assay components to a solid phase. These include, without limitation, marker or probe molecules which are immobilized through conjugation of biotin and streptavidin. Such biotinylated assay components can be prepared from biotin-NHS(N-hydroxy-succinimide) using techniques known in the art (e.g., biotinylation kit, Pierce Chemicals, Rockford, IL), and immobilized in the wells of streptavidin-coated 96 well plates (Pierce Chemical). In certain descriptions, the surfaces with immobilized assay components can be prepared in advance and stored.

Other suitable carriers or solid phase supports for such assays include any material capable of binding the class of molecule to which the marker or probe belongs. Well known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilized component is added to the solid phase upon which the second component is anchored. After the reaction is complete, uncomplexed components may be removed (e.g., by washing) under conditions such that any complexes formed will remain immobilized upon the solid phase. The detection of marker/probe complexes anchored to the solid phase can be accomplished in a number of methods outlined herein.

In certain exemplary descriptions, the probe, when it is the unanchored assay component, can be labeled for the purpose of detection and readout of the assay, either directly or indirectly, with detectable labels discussed herein and which are well-known to one skilled in the art.

It is also possible to directly detect marker/probe complex formation without further manipulation or labeling of either component (marker or probe), for example by utilizing the technique of fluorescence energy transfer (see, for example, U.S. Patent Nos. 5,631,169 and 4,868, 103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another description, determination of the ability of a probe to recognize a marker can be accomplished without labeling either assay component (probe or marker) by utilizing a technology such as real-time Biomolecular Interaction Analysis (BIA) (see, e.g., Sjolander, S. and Urbaniczky, C, 1991, Anal. Chem. 63:2338 2345 and Szabo et al, 1995, Curr. Opin. Struct. Biol. 5:699 705). As used herein, "BIA" or "surface plasmon resonance" is a technology for studying biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another description, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas and Minton (1993) Trends Biochem. Sci. 18:284). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, e.g., Heegaard (1998) J. Mol. Recognit. 11 : 141 ; Hage and Tweed (1997) J. Chromatogr. B. Biomed. Sci. Appl. 12:499). Gel electrophoresis may also be employed to separate complexed assay components from unbound components (see, e.g., Ausubel et al, ed., Current Protocols in Molecular Biology, John Wiley & Sons, New York, 1987 1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

In certain exemplary descriptions, the level of mRNA corresponding to the marker can be determined either by in situ and/or by in vitro formats in a biological sample using methods known in the art. Many expression detection methods use isolated RNA. For in vitro methods, any RNA isolation technique that does not select against the isolation of mRNA can be utilized for the purification of RNA from blood cells (see, e.g., Ausubel et al, ed., Current Protocols in Molecular Biology, John Wiley & Sons, New York 1987 1999). Additionally, large numbers of cells and/or samples can readily be processed using techniques well known to those of skill in the art, such as, for example, the single- step RNA isolation process of Chomczynski (1989, U.S. Patent No. 4,843,155).

Isolated mRNA can be used in hybridization or amplification assays that include, but are not limited to, Southern or Northern analyses, polymerase chain reaction analyses and probe arrays. In certain exemplary descriptions, a diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to an mRNA or genomic DNA encoding a marker of the present description. Other suitable probes for use in the diagnostic assays of the description are described herein. Hybridization of an mRNA with the probe indicates that the marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in a gene chip array. A skilled artisan can readily adapt known mRNA detection methods for use in detecting the level of mRNA encoded by the markers of the present description.

An alternative method for determining the level of mRNA corresponding to a marker of the present description in a sample involves the process of nucleic acid amplification, e.g., by RT-PCR (the experimental embodiment set forth in U.S. Patent Nos. 4,683,195 and 4,683,202), COLD-PCR (Li et al. (2008) Nat. Med. 14:579), ligase chain reaction (Barany, 1991, Proc. Natl. Acad. Sci. USA, 88: 189), self sustained sequence replication (Guatelli et al., 1990, Proc. Natl. Acad. Sci. USA 87: 1874), transcriptional amplification system (Kwoh et al. (1989) Proc. Natl. Acad. Sci. USA 86: 1173), Q- Beta Replicase (Lizardi et al. (1988) Bio/Technology 6: 1 197), rolling circle replication (U.S. Patent No.5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5 or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For in situ methods, mRNA does not need to be isolated from the sample (e.g., a bodily fluid (e.g., blood cells)) prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, e.g., a housekeeping gene that is constitutively expressed. Suitable genes for normalization include housekeeping genes such as the actin gene, or epithelial cell-specific genes. This normalization allows the comparison of the expression level in a patient sample from one source to a patient sample from another source, e.g., to compare a >2n phagocytic blood cell from an individual to a =2n phagocytic blood cell from the individual.

In one description, a protein or polypeptide corresponding to a marker is detected. In certain descriptions, an agent for detecting a protein or polypeptide can be an antibody capable of binding to the polypeptide, such as an antibody with a detectable label. As used herein, the term "labeled," with regard to a probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. Antibodies can be polyclonal or monoclonal. An intact antibody, or a fragment thereof (e.g., Fab or F(ab')2) can be used. In one format, antibodies, or antibody fragments, can be used in methods such as Western blots or immunofluorescence techniques to detect the expressed proteins. In such uses, it is generally preferable to immobilize either the antibody or proteins on a solid support. Suitable solid phase supports or carriers include any support capable of binding an antigen or an antibody. Well known supports or carriers include glass, polystyrene, polypropylene, polyethylene, dextran, nylon, amylases, natural and modified celluloses, polyacrylamides, gabbros, magnetite and the like.

A variety of formats can be employed to determine whether a sample contains a protein that binds to a given antibody. Examples of such formats include, but are not limited to, competitive and non-competitive immunoassay, enzyme immunoassay (EIA), radioimmunoassay (RIA), antigen capture assays, two-antibody sandwich assays, Western blot analysis, enzyme linked immunoabsorbant assay (ELISA), a planar array, a colorimetric assay, a chemiluminescent assay, a fluorescent assay, and the like. Immunoassays, including radioimmmunoassays and enzyme- linked immunoassays, are useful in the methods of the present description. A skilled artisan can readily adapt known protein/antibody detection methods for use in determining whether cells (e.g., bodily fluid cells such as blood cells) express a marker of the present description.

One skilled in the art will know many other suitable carriers for binding antibody or antigen, and will be able to adapt such support for use with the present descriptions. For example, protein isolated from cells (e.g., bodily fluid cells such as blood cells) can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In certain exemplary descriptions, assays are provided for diagnosis, prognosis, assessing the risk of developing a disease, assessing the efficacy of a treatment, monitoring the progression or regression of a disease, and identifying a compound capable of ameliorating or treating a disease. An exemplary method for these methods involves obtaining a bodily fluid sample from a test subject and contacting the bodily fluid sample with a compound or an agent capable of detecting one or more of the markers of the disease or condition, e.g., marker nucleic acid (e.g., mRNA, genomic DNA), marker peptide (e.g., polypeptide or protein), marker lipid (e.g., cholesterol), or marker metabolite (e.g., creatinine) such that the presence of the marker is detected in the biological sample. In one description, an agent for detecting marker mRNA or genomic DNA is a labeled nucleic acid probe capable of hybridizing to marker mRNA or genomic DNA. The nucleic acid probe can be, for example, a full-length marker nucleic acid or a portion thereof. Other suitable probes for use in the diagnostic assays of the description are described herein.

As used herein, a compound capable of ameliorating or treating a disease or condition can include, without limitations, any substance that can improve symptoms or prognosis, prevent progression of the disease or condition, promote regression of the disease or condition, or eliminate the disease or condition.

Also described is a method for identifying a compound capable of ameliorating or treating a disease or condition in a subject comprising: a) determining a first profile of one or more markers of the disease or condition from a population of >2n phagocytic cells from the subject before administering the compound to the subject; determining a second profile of at least one of the one or more markers from a population of =2n phagocytic cells from the subject before administering the compound to the subject; identifying a first difference between the first and second profiles of at least one or more of said markers; b) determining a third profile of the one or more markers from a population of >2n phagocytic cells from the subject after the administration of the compound; determining a fourth profile of at least one of the one or more markers from a population of =2n phagocytic cells from the subject after the administration of the compound; identifying a second difference between the third and fourth profiles of at least one or more of said markers; c) identifying a difference between the first difference and the second difference, wherein the identified difference indicates that the compound is capable of ameliorating or treating said disease or condition in the subject.

Also described is a method for identifying one or more markers of a disease or condition comprising: a) determining a first profile of analytes from >2n phagocytic cells from a subject having said disease or condition; determining a second profile of analytes from =2n phagocytic cells from the subject having said disease or condition; identifying a first set of differences between the first and second profiles, wherein the first set of differences is specific to the first profile relative to the second profile; b) determining a third profile of analytes from >2n phagocytic cells from a control subject not having said disease or condition; determining a fourth profile of analytes from =2n phagocytic cells from the control subject not having said disease or condition; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises: d) obtaining a fifth profile of analytes from cells or tissues affected by said disease or condition from the subject having said disease or condition; obtaining a sixth profile of analytes from cells or tissues not affected by said disease or condition from the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

An exemplary method for detecting the presence or absence of an analyte (e.g., DNA, RNA, protein, polypeptide, carbohydrate, lipid or the like) corresponding to a marker of the description in a biological sample involves obtaining a bodily fluid sample (e.g., blood) from a test subject and contacting the bodily fluid sample with a compound or an agent capable of detecting one or more markers. Detection methods described herein can be used to detect one or more markers in a biological sample in vitro as well as in vivo. For example, in vitro techniques for detection of mR A include Northern hybridizations and in situ hybridizations. In vitro techniques for detection of a polypeptide corresponding to a marker of the description include enzyme linked immunosorbent assays (ELISAs), Western blots, immunoprecipitations and immunofluorescence. In vitro techniques for detection of genomic DNA include Southern hybridizations. Furthermore, in vivo techniques for detection of a polypeptide corresponding to a marker of the description include introducing into a subject a labeled antibody directed against the polypeptide. For example, the antibody can be labeled with a radioactive marker whose presence and location in a subject can be detected by standard imaging techniques. Because each marker is also an analyte, any method described herein to detect the presence or absence of a marker can also be used to detect the presence or absence of an analyte.

The marker that is useful in the methods described can include any mutation in any one of the above-identified markers. Mutation sites and sequences can be identified, for example, by databases or repositories of such information, e.g., The Human Gene Mutation Database (www.hgmd.cf.ac.uk), the Single Nucleotide Polymorphism Database (dbSNP, www.ncbi.nlm.nih.gov/projects/SNP), and the Online Mendelian Inheritance in Man (OMIM) website (www.ncbi.nlm.nih.gov/omim).

The marker that is useful in the methods described can include any marker that is known to be associated with a disease or condition. Markers that can be used can be any marker that has been well-characterized as associated with a specific disease or condition, or any markers that have been identified by the methods described.

In some descriptions, the markers comprise at least one gene selected from the group consisting of AKT2, BA 1, EGFR, ERBB2, ETS2, FOS, JUN, MAP2K1, MMP2, PDGFB, RB I, SERPINB2, SNCG, and SPP1. In some descriptions, the one or more markers comprise at least one gene selected from the group consisting of AKT1, AKT2, BAK2, CDC25A, E2F1, EGFR, ERBB2, FOS, JUN, MAP2K1, MMP2, NFKBl, PDGFB, PIK3R1, PNN, RBI, SERPINB2, SERPINB5, SNCG, SPP1, TERT, TIMP3, and TP53. In some descriptions, the one or more markers comprise at least one gene selected from the group consisting of CASP8, CASP9, COL18A1, ETS2, HTATIP2, MMP9, SRC, and TWIST 1. In some descriptions, the one or more markers comprise at least one gene selected from the group consisting of AKT1, APAF 1, ATM, CDC25A, CDKN1A, ETS2, FOS, IL8, ITGA4, ITGA6, ITGAV, JUN, MAP2K1, NFKBIA, PLAU, PLAUR, RAF1, SERPTNB2, SYK, TIMP1, TNF, TNFRSFIOB, and TNFRSF 1A. In some descriptions, the markers comprise at least one gene selected from the group consisting of ACP2, AK2, AKT3, ARL5B, ATP2B3, BGN, BRAF, BTG2, CAMKK2, CAPG, CAPN12, CPLX2, DENND5A, DNA2, FAM104A, FNIP 1, GFRA4, GLUD1, GNAQ, GP1BB, HNRPLL, HOXA2, HPS3, ΓNPP4A, ITGAV, KLHL23, LANCL2, LYPD6, MAPKAPK3, MEF2A (includes, EG:4205), MEF2C, NVL, PCYT1A, PGLYRP4, PLOD1, PPP1CB, PRKAB2, PROS1, PTPRE, RASA4 (includes,EG: 10156), RBMS2, RBPI, STAT5B, THBS1, TRIB1, TRIM2, TSPAN6, and ZDHHC21. In some descriptions, the markers comprise at least one gene selected from the group consisting of B4GALT5, BOP1, CCL2, CCL3, CCL3L1, CCRL2, CD83, CLEC4G, CLIC4, CTSC, CTSO, CXCL10, FCGR3A, FPR3, HBA1, HBB, LRMP, MAP1LC3B2, MS4A4A, MSR1, MYADML, NIDI, PF4, PION, RNF217, SAMD9L, SERPING1, and SPARC. In some descriptions, the markers comprise at least one gene selected from the group consisting of ACOT9, AMPD2, ARHGAP15, BATF2, C3AR1, C5orf41, CCL3, CCL3L1, CD63, CHST11, CHSY1, CLEC4G, CTSZ, CXorf21, CYTH4, CYTIP, DLEU2, DNAJA1, DOCK8, DTX3L, DUSP6, EPSTI1, ERF, F2RL1, FYB, GABRB2, GBP 5, GLRX, GNB4, ICAM1, IFI35, IFIH1, IFNAR2, IL1R1, IRF1, ITGA5, LAP3, LAPTM5, LCP2, MAP I LOB, MAP1LC3B2, MICAL2, MT1DP, MT1JP, MT1M, MT2A, MYADML, NEK6, NLNJ2, NNMT, NT5C3L, NUBl, PDE4B, PLODl, PML, PRKCB, PSMB9, RCN3, RGS4, RNASE6, RTP4, SAMD9L, SEL1L, SERPINGl, SETX, SIGLEC10, SKIL, SLC7A7, SNORA21, SP100, SP 110, SP140, SSFA2, STAT2, STK17B, STK3, TDRD7, TMCC1, TMPRSS1 1E2, TNFRSF 1B, TPM1, TRIM21,TXNDC4, UBE2L6, UBE2W, USP 18, VAV1, WARS, WIPF1, and WIPI1. In some descriptions, the markers comprise at least one gene selected from the group consisting of ADAR, ADM, ALAS1, ANKRD22, ARHGAP27, B3GNT5, BCL10, C12orf35, C15orf29, C2orf59, CD177, CEACAM1, CPEB2, DDX58, F2RL1, GDPD3, GNAI3, HIST2H3A, HIST2H3D, HIST2H4A, HMGCR, HSPA6, HSPC159, IL4R, IMPA2, KPNB1, KREMEN1, KRT23, LDLR, LOC100130904, LTB4R, MAEA, MARK2, MBOAT2, MPZL3, N4BP 1, NBEAL2, NMI, NPEPPS, PARP 14, PGM2, PPIF, PXN, RALBP1, R0D1, RPS6KA1, S100P, SERTAD2, SLC9A1, SLPI, SP110, SPINT1, ST14, TBC1D3, TNFRSF9, TRIM21, UPP1, VPS24, ZBTB34, and ZNF256.

In some descriptions, the marker that is useful in the methods described for prenatal or pregnancy-related diseases or conditions include those disclosed in, for example, United States Patents 7,655,399, 7,651,838, 6,660,477, 6,172, 198, 5,594,637, 5,514,598, 6,258,540, 6,664,056, 7,235,359, and 7,645,576, United States Patent Application Publications 20090162842, 20090155776, 20070207466, 20060019278, 20040086864, 20020045176, 20010051341, 20020192642, 20040009518, 20040203037, 20050282185, 20060252071, 20070275402, 20080153090, 20090170102, 20090061425, 20020045176, 20040137452, 20050164241, 20060019278, 20060252068, 20060252071, 20060257901, 20070141625, 20070218469, 20070275402, 20090155776, 20090162842, 20090170102, 20090317797, 20100120056, 20100120076, and 20100137263 and International Patent Application Publications WO/2006/026020, WO/2002/068685, WO/2005/111626, WO/2009/055487, WO/2009/001392, and WO/2008/014516.

In some descriptions, the marker that is useful in the methods described for neurological or neuropsychiatric diseases or conditions include those disclosed in, for example in United States Patents 7,723, 117, 6,867,236, United States Patent Application Publications 20060115854, 20060115855, 20060166283, 20060234301, 20060259990, 20060259991, 20070162983, 20070264197, 20080026405, 20080038730, 20080051334, 20080152589, 20080220013, 20080261226, 20080269103, 20080286263, 20090041862, 20090239241, 20090275046, 20090318354, 20090324611, 20100009352, 20100021929, 20100028356, 20100055722, 20100062463, 20100075891, 20100105623, 20100124756, 20100159486, 20100167937, 20100169988, 20100167320, 20100112587, 20100098705, 20100068705, 20100009356, 20090305265, 20100124746, 20100092983, 20070148661, 20070141625, 20100120050, 20090155230, 20090274709, International Patent Application Publications WO/2004/040016, WO/2004/071269, WO/2005/033341, WO/2005/052592, WO/2005/103712, WO/2005/114222, WO/2006/020269, WO/2006/048778, WO/2006/050475, WO/2006/061609, WO/2006/105907, WO/2006/133423, WO/2006/134390, WO/2007/098585, WO/2007/1 19179, WO/2008/010660, WO/2008/014314, WO/2008/028257, WO/2008/046509, WO/2008/046 10, WO/2008/046511, WO/2008/046512, WO/2008/063369, WO/2008/085035, WO/2008/095261, WO/2008/100596, WO/2008/120684, WO/2008/125651, WO/2008/127317, WO/2008/132464, WO/2009/000520, WO/2009/001392, WO/2009/068591, WO/2009/074331, WO/2009/100131, WO/2010/005750, WO/2010/011506, WO/2010/019553, WO/2010/059242, WO/2010/061283, WO/2010/063009, WO/2010/066000, WO/2009/121 152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/07556.

In some descriptions, the marker that is useful in the methods described for cardiovascular diseases or conditions include those disclosed in, for example in United States Patents 7,670,769, 7,445,886, 7,432, 107, 7,157,235, and 7,009,038, United States Patent Application Publications 20100167320, 20100112587, 20100098705, 20100068705, 20100009356, 20090305265, 20100124746, 20100092983, 20070148661, 20070141625, 20100120050, 20090155230, and 20090274709, and International Patent Application Publications WO/2009/121152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/075566.

In some descriptions, the marker that is useful in the methods described for kidney-associated diseases or conditions include those disclosed in, for example in United States Patents 7,488,584, 7,459,280, 7,294,465, and 7,662,578, United States Patent Application Publications 20100143951, 20100124746, 20100120056, 20100120041, 20100081142, 20090155230, and 20090239242, International Patent Application Publications WO/2010/059996, WO/2010/054389, WO/2010/048347, WO/2010/048497, WO/2010/054167, WO/2010/048346, WO/2010/046137, WO/2010/025434, WO/2010/018185, WO/2010/012306, WO/2009/122387, WO/2009/083950, WO/2009/080780, WO/2009/060035, WO/2009/059259, WO/2008/154238, WO/2008/089936, WO/2008/084331, WO/2008/042012, WO/2007/131345, WO/2005/012907, WO/2004/024098, WO/2003/019193, WO/2007/1 12999, WO/2007/082733, WO/2006/073941, WO/2010/068686, WO/2010/022210, and WO/2009/127644.

In some descriptions, the marker that is useful in the methods described for autoimmune or immune-related diseases or conditions include those disclosed in, for example 7,604,948, 7,670,764, 6,986,995, and 6,631,330, United States Patent Application Publication 20070141625, 20090263474, 20100075891, 20100104579, 20100105086, 20100131286, 20090176217, 20090202469, 20020119118, 20090258025, 20100137393, 20100120629, 20090318392, 20090196927, 20090023166, 20080227709, 20080039402, 20080026378, 20070224638, 20070218519, 20060210562, 20050266432, 20050164233, 20050130245, 20090130683, 200901 10667, 20090054321, 20090023166, and 20080274118, and International Patent Application Publication WO/2009/043848, WO/2010/053587, WO/2010/046503, WO/2010/039714, WO/2009/100342, WO/2009/053537, WO/2009/017444, WO/2008/156867, WO/2008/147938, WO/2008/129296, WO/2008/137835, WO/2008/082519, WO/2008/064336, WO/2008/043782, WO/2008/043725, WO/2007/047907, WO/2006/125117, WO/2006/1 14661, WO/2006/020899, WO/2005/1 14222, WO/2005/007836, WO/2004/076639, WO/2004/050704, and WO/2001/014881.

Also described are kits that comprise marker detection agents that detect at least one or more of the markers identified by the methods described. Also described are methods of treating or preventing a disease or condition in a subject comprising administering to said subject an agent that modulates the activity or expression of at least one or more of the markers identified by the methods described.

### Examples

### Example 1: Sorting of White Blood Cells into Phagocytic Cells with a DNA content of 2n and Phagocytic Cells with a DNA content >2n

White blood cells were isolated from human blood (donor) stained with Hoechst 33342, and sorted by FACS. FIG. 7 shows that this approach is capable of identifying, sorting, and collecting 10⁶ white blood cells of each of the two desired phagocytic cell populations.

### Example 2: A Representative Method for the Separation of Phagocytic Cells and the Analysis of Expression Profiles

Stain white blood cells with fluorescent antibodies specific against one or more phagocytic cells (e.g., neutrophils, macrophages, or monocytes) and then stain with DNA-binding dye (e.g., propidium iodide).

Sort the cells (FACS) into 2n and >2n phagocytes.

Isolate NA from each of the 2n and >2n phagocytes. Prepare cDNA, cRNA and use to differentiate genetic profiles (e.g., cancer gene array) of 2n- phagocytic and >2n-phagocytic cells.

Isolate DNA from each of the 2n and >2n phagocytes. Run DNA arrays and compare the profiles obtained from 2n-phagocytic and >2n-phagocytic cells.

Isolate protein from each of the 2n and >2n phagocytes. Run Western blots using antibodies to known proteins overexpressed by human tumors (e.g., PSA and PSMA in prostate cancer; CEA in colon cancer; and CA125 in ovarian cancer), and compare the profiles obtained from 2n-phagocytic and >2n- phagocytic cells.

Isolate lipids from each of the 2n and >2n phagocytes. Compare quantity and quality of lipids, for example using HPLC.

### Example 3: Profiling Experiments

### Isolation of Blood Phagocytic Cells

A sample of blood is obtained from a patient. The blood (∼5 mL) will be transferred to a 50-mL tube containing 50 µL 0.5 M EDTA (final EDTA concentration = ∼4.8 mM). The tube will be vortexed gently and 25 mL BC Lysis Buffer (Norgen, Incorporated) will be added. The tube will be vortexed gently again, incubated at room temperature until the color of the solution changes to bright red (3-5 min), and centrifuged at 2,000 rpm for 3 min. Following careful aspiration of the supernatant, the WBCs will be washed with 40 mL Ca/Mg-free 0.1 M PBS (containing 2% FBS, 2 mM EDTA, and 20 mM glucose), and the cells (106/mL) will then be incubated (30 min, 4 0C, in the dark) with a cell-staining solution containing (i) the DNA, viable cell-permeable stain Hoechst 33342 (4 µg/mL; Em = 483 nm), (ii) the anti-human monocytes/macrophages monoclonal antibody (Alexa Fluor® 647-conjugate; Em =668 nm), which recognizes the human F4/80 antigen expressed by circulating monocytes/macrophages, and (iii) the anti-human neutrophil monoclonal antibody (RPE-conjugate; Em = 578 nm), which recognizes human circulating neutrophils. The cells will then be washed and sorted (BD FACSAria) into neutrophils (Nₙ₌₂), neutrophils (Nₙ>2), monocytes/macrophages (M/Mₙ₌₂), and monocytes/macrophages (M/M_{n>2}).

### Gene Profiling

Human whole-genome gene profiling will be performed. For RNA samples obtained from human tumor cells or neutrophils (Nn=2, Nn>2) and monocytes/macrophages (M/Mn=2, M/Mn>2), the GeneChip® Human Genome Ul 33 Plus 2.0 Array by Affymetrix, Incorporated will be used. This array analyzes the expression level of over 47,000 transcripts and variants, including 38,500 well-characterized human genes. In general, the extracted R A will be used to determine the expression profiles of human genes using the above-mentioned array. To ensure array reproducibility, each sample will be profiled in triplicate and the experiment repeated once. The microarray data will be filtered for cancer- induction-related genes as described below and validated using quantitative real- time, reverse transcriptase, polymerase chain reaction (RT-PCR).

### Upregulation/Downregulation of Cancer-Induction-Related Genes

RNA will be isolated using Triazol (Invitrogen, Incorporated) and purified using the cartridges provided in the kit. The RNA quality and quantity will be assessed with the Bioanalyzer 2100 (Agilent Technologies, Incorporated, Palo Alto, CA) and Degradometer software version 1.41 (Worldwide Web: dnaarrays.org). These experimental results will help in distinguishing the molecular pathways perturbed consequent to the presence of tumors.

### Analysis of Microarray Experiments

The analysis of the large scale/high throughput molecular expression data generated will rely heavily on the ability to (i) identify genes differentially expressed in phagocytic cells with a DNA content >2, (ii) annotate the identified genes, and (iii) assign the annotated genes to those specifically expressed by a specific tumors. Statistical analysis of the microarray data can be done, for example, using the dChip package which easily accommodates this type of gene list construction in its "Analysis/Compare Samples" menu. When using Affymetrix GeneChips, one or more Gene Chips and associated methods will be applied to ascertain the quality of the raw microarray data (Gautier et al. (2004) Bioinformatics 20:307). Furthermore, various background correction and normalization procedures will be utilized to arrive at an optimal protocol for normalization and summarization of the probe sets (to produce expression values) (Huber et al. (2002) Bioinformatics 18(Suppl. 1):S96; Wu et al. (2004) Journal of the American Statistical Association 99:909; Seo and Hoffman (2006) BioMed Central Bioinformatics 7:395). In a two-step filtration approach, we will compare the gene profiles of Pₙ₌₂ to those of P_{n>2} and construct a list of expressed genes and then compare these genes to the tumor-specific genes identified for each tumor cell line - post filtration of Pₙ₌₂ gene profile as shown in FIG. 5. For example, (i) blood will be obtained from breast cancer patients; (ii) neutrophils (n>2 and n=2) will be isolated and their gene profiles determined in triplicate; (iii) the mean (from the 3 samples) of each identified gene and its respective standard error (SE) will be calculated for each group (N_{n>2} and Nₙ₌₂); (iv) the gene expression profiles of the two groups will then be compared and a list (L-I) of expressed genes identified on the basis of an absolute >2-fold log change (N_{n>2}/Nₙ₌₂), according to the Welch modified two-sample t-test; (v) the gene expression profiles of Nₙ₌₂ and that of breast cancer (obtained from tumor and normal breast tissue biopsies) will be compared and a list (L-2) of expressed genes identified; and (vi) breast-cancer- specific gene signatures that have been acquired/expressed by N_{n>2} will be identified by comparing the genes in L-I and L-2 ("Analysis/Compare Samples/Combine Comparisons," dChip) and filtering common genes.

### Protein Profiling

Fifty to one hundred micrograms of the total protein from each type of cells will be denatured and reduced with tris-(2-carboxyethyl)phosphinetrypsin (1 mM) and 0.02% sodium dodecyl sulfate at 60 0C for 1 hour. Cysteines are subsequently blocked and total protein is digested with trypsin at 37 0C for 12-16 hours. The resulting peptides will be iTRAQ-labeled (with tags 113-1 19 and 121) for 1 hour (4-plex or 8-plex depending on the number of cell types to be compared). Following labeling, the separately tagged samples are combined and injected into an Agilent 1200 Series HPLC system equipped with a strong cation exchange column (Applied Biosystems 4.6 x 100 Porous). The 96 collected fractions are then pooled into 14 fractions, and each fraction is injected into the LC Packings Ultimate HPLC System for a second round of fractionation under reverse-phase conditions (LC Packings 15 cm x 75 µm analytical column). The reverse-phase fractions are spotted directly onto the target plate using an LC Packings Probot and are analyzed with mass spectrometry (Applied Biosystems 4800 Plus Proteomics Analyzer). Following data acquisition, the spectra are processed using the ProteinPilot software package (Applied Biosystems MDS Sciex), and the individual proteins in each of the cell types with their relative expression levels are identified using the ProteinPilot™ software.

## Claims

1. A method for monitoring the progression or regression of cancer in a subject comprising:
a) preparing a first nucleic acid and/or protein profile of one or more markers of cancer by detecting the one or more markers from a population of the subject's >2n phagocytic cells at a first time point;
preparing a second nucleic acid and/or protein profile of at least one of the one or more markers by detecting at least one of the one or more markers from a population of the subject's =2n phagocytic cells at the first time point;
identifying a first difference between the first and second profiles of at least one or more of said markers;
b) preparing a third nucleic acid and/or protein profile of the one or more markers by detecting the one or more markers from a population of the subject's >2n phagocytic cells at a second time point;
preparing a fourth nucleic acid and/or protein profile of at least one of the one or more markers by detecting at least one of the one or more markers from a population of the subject's =2n phagocytic cells at the second time point;
identifying a second difference between the third and fourth profiles of at least one or more of said markers; and
c) identifying a difference between the first difference and the second difference, wherein the identified difference is indicative of the progression or regression of said cancer in the subject.

2. The method of claim 1, wherein the profile is a nucleic acid profile, a protein profile, or a combination thereof;
optionally wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof; and
optionally wherein the protein profile is a protein expression profile, a protein activation profile, or a combination thereof; and further optionally wherein the protein activation profile comprises determining a phosphorylation state, an ubiquitination state, a myristoylation state, or a conformational state of the one or more markers.

3. The method of any one of the claims 1 or 2, wherein the one or more markers are nucleic acids, proteins, or combinations thereof;
optionally wherein the nucleic acids are nucleotides, oligonucleotides, DNAs, RNAs, or DNA-RNA hybrids; and
optionally wherein the proteins are amino acids, peptides, enzymes, antigens, antibodies, cytokines, lipoproteins, glycoproteins, or hormones.

4. The method of any one of claims 2 or 3, wherein the analytes are nucleic acids, proteins, or combinations thereof;
optionally wherein the nucleic acids are nucleotides, oligonucleotides, DNAs, RNAs, or DNA-RNA hybrids;
optionally wherein the proteins are amino acids, peptides, enzymes, antigens, antibodies, cytokines, lipoproteins, glycoproteins, or hormones.

5. The method of any one of the claims 1-4, wherein the profile is a nucleic acid profile, a protein profile, or a combination thereof;
optionally wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof; and
optionally wherein the protein profile is a protein expression profile, a protein activation profile, or a combination thereof; and further optionally wherein the protein activation profile comprises determining a phosphorylation state, an ubiquitination state, a myristoylation state, or a conformational state of the one or more markers.

6. The method of any one of claims 1-5, wherein the profile is determined by a qualitative assay, a quantitative assay, or a combination thereof;
optionally wherein the quantitative assay uses sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLID® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.

7. The method of any one of claims 1-5, wherein the profile is a nucleic acid profile detected by polymerase chain reaction (PCR) analysis, sequencing analysis, electrophoretic analysis, restriction fragment length polymorphism (RFLP) analysis, Northern blot analysis, reverse-transcriptase-PCR analysis (RT-PCR), quantitative PCR, quantitative RT-PCR, allele-specific oligonucleotide hybridization analysis, comparative genomic hybridization, heteroduplex mobility assay (HMA), single strand conformational polymorphism (SSCP), denaturing gradient gel electrophoresis (DGGE), RNAase mismatch analysis, mass spectrometry, mass spectrometry, matrix assisted laser desorption/ionization-time of flight (MALDI-TOF) mass spectrometry, electrospray ionization (ESI) mass spectrometry, surface-enhanced laser desorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, surface plasmon resonance, bisulfite-driven conversion of non-methylated cytosine to uracil, methyl-binding PCR analysis, or a combination thereof;
optionally wherein the sequencing analysis is selected from the group consisting of direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof;
or wherein the profile is a protein profile detected by an immunohistochemistry assay, an enzyme-linked immunosorbent assay (ELISA), chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser desorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, surface plasmon resonance, microfluidic chip-based assays, Western blotting assay, or a combination thereof.

8. The method of any one of the claims 1 or 2, further comprising extracting cellular contents from the >2n phagocytic cells or the =2n phagocytic cells before a).

9. The method of any one of claims 1 or 2, wherein the >2n phagocytic cells or the =2n phagocytic cells are professional phagocytic cells, non-professional phagocytic cells, or mixtures thereof;
optionally wherein the professional phagocytic cells are neutrophils, macrophages, monocytes, dendritic cells, foam cells, mast cells, eosinophils, or mixtures thereof;
optionally wherein the non-professional phagocytic cells are epithelial cells, endothelial cells, fibroblasts, mesenchymal cells, or mixtures thereof; and
optionally wherein the >2n phagocytic cells or the =2n phagocytic cells or the non- phagocytic cells are isolated from a bodily fluid sample, tissues, or cells of the subject; optionally wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, or ocular fluid.

## Patentansprüche

1. Verfahren zum Überwachen des Fortschreitens oder Rückgangs von Krebs bei einem Patienten, Folgendes umfassend:
a) Herstellen eines ersten Nukleinsäure- und/ oder eines Proteinprofils eines oder mehrerer Marker von Krebs durch Detektieren des einen oder mehrerer Marker aus einer Population der >2n phagozytischen Zellen des Patienten zu einem ersten Zeitpunkt;
Herstellen eines zweiten Nukleinsäure- und/ oder eines Proteinprofils mindestens eines von dem einen oder mehrerer Marker durch Detektieren mindestestens eines von dem einen oder mehrerer Marker aus einer Population der =2n phagozytischen Zellen des Patienten zu einem ersten Zeitpunkt;
Identifizieren einer ersten Differenz zwischen dem ersten und zweiten Profil von mindestens einem oder mehreren der Marker;
b) Herstellen eines dritten Nukleinsäure- und/ oder eines Proteinprofils des einen oder mehrerer Marker durch Detektieren des einen oder mehrerer Marker aus einer Population der >2n phagozytischen Zellen des Patienten zu einem zweiten Zeitpunkt;
Herstellen eines vierten Nukleinsäure- und/ oder eines Proteinprofils mindestens eines von dem einen oder mehrerer Marker durch Detektieren mindestestens eines von dem einen oder mehrerer Marker aus einer Population der =2n phagozytischen Zellen des Patienten zu einem zweiten Zeitpunkt;
Identifizieren einer zweiten Differenz zwischen dem dritten und vierten Profil von mindestens einem oder mehreren der Marker; und
c) Identifizieren einer Differenz zwischen der ersten Differenz und der zweiten Differenz, wobei die identifizierte Differenz auf das Fortschreiten oder den Rückgang des Krebses bei dem Patienten hinweist.

2. Verfahren nach Anspruch 1, wobei das Profil ein Nukleinsäureprofil, ein Proteinprofil, oder eine Kombination davon ist;
wobei das Nukleinsäureprofil optional ein gentypisches Profil, ein Single Nucleotide Polymorphism Profil, ein Genmutationsprofil, ein Genkopienzahlprofil, ein DNS-Methylierungsprofil, ein DNS-Acetylierungsprofil, ein Chromosomendosierprofil, ein Genexpressionsprofil oder eine Kombination davon ist; und
wobei das Proteinprofil optional ein Proteinexpressionsprofil, ein Proteinaktivierungsprofil, oder eine Kombination davon ist; und wobei das Proteinaktivierungsprofil weiter optional das Bestimmen eines Phosphorylierungszustands, eines Ubiquitinierungszustands, eines Myristoylierungszustands oder eines konformativen Zustands des einen oder mehrerer Marker umfasst.

3. Verfahren nach einem der Ansprüche 1 oder 2, wobei der eine oder mehrere Marker Nukleinsäuren, Proteine oder Kombinationen davon sind;
wobei die Nukleinsäuren optional Nukleotide, Oligonukleotide, DNS, RNS, oder DNS-RNS-Hybride sind; und
wobei die Proteine optional Aminosäuren, Peptide, Enzyme, Antigene, Antikörper, Zytokine, Lipoproteine, Glykoproteine oder Hormone sind.

4. Verfahren nach einem der Ansprüche 2 oder 3, wobei die Analyte Nukleinsäuren, Proteine, oder Kombinationen davon sind;
wobei die Nukleinsäuren optional Nukleotide, Oligonukleotide, DNS, RNS, oder DNS-RNS-Hybride sind; und
wobei die Proteine optional Aminosäuren, Peptide, Enzyme, Antigene, Antikörper, Zytokine, Lipoproteine, Glykoproteine oder Hormone sind.

5. Verfahren nach einem der Ansprüche 1 bis 4, wobei das Profil ein Nukleinsäureprofil, ein Proteinprofil oder eine Kombination davon ist;
wobei das Nukleinsäureprofil optional ein gentypisches Profil, ein Single Nucleotide Polymorphism Profil, ein Genmutationsprofil, ein Genkopienzahlprofil, ein DNS-Methylierungsprofil, ein DNS-Acetylierungsprofil, ein Chromosomendosierprofil, ein Genexpressionsprofil oder eine Kombination davon ist; und
wobei das Proteinprofil optional ein Proteinexpressionsprofil, ein Proteinaktivierungsprofil, oder eine Kombination davon ist; und wobei das Proteinaktivierungsprofil weiter optional das Bestimmen eines Phosphorylierungszustands, eines Ubiquitinierungszustands, eines Myristoylierungszustands oder eines konformativen Zustands des einen oder mehrerer Marker umfasst.

6. Verfahren nach einem der Ansprüche 1 bis 5, wobei das Profil durch ein qualitatives Assay, ein quantitatives Assay oder eine Kombination davon bestimmt wird;
wobei das quantitative Assay optional eine Sequenzierung, direkte Sequenzierung, Schrotschuss Sequenzierung, Dideoxy-Sequenzierung nach Sanger, Sequenzierung von Gesamtgenomen, Sequenzierung durch Hybridisierung, Pyrosequenzierung, Kapillarelektrophorese, Gelelektrophorese, Duplex-Sequenzierung, Zyklus-Sequenzierung, Einzelbasenextensionssequenzierung, Festphasen-Sequenzierung, Hochdurchsatz-Sequenzierung, Massively Parallel Signature Sequenzierung, Emulsion PCR, Sequenzierung durch reversiblen Farbstoffterminator, Paired-End Sequenzierung, Near-Term Sequenzierung, Exonuklease-Sequenzierung, Sequenizerung durch Ligatur, Short-Read Sequenzierung, Einzelmolekül-Sequenzierung, Sequenzierung durch Synthese, Echtzeit-Sequenzierung, Reverse-Terminator Sequenzierung, Nanoporen Sequenzierung, 454 Sequenzierung, Solexa Genome Analyzer Sequenzierung, SOLID® Sequenzierung, MS-PET Sequenzierung, Massenspektrometrie, Matrix-assistierte-Laserdesorption/Ionisations-Flugzeit (MALDI-TOF) Massenspektrometrie, Elektrospraylonisation (ESI) Massenspektrometrie, oberflächenverbesserte Laserdesorption/Ionisations-Flugzeit (SELDI-TOF) Massenspektrometrie, Quadrupel-Flugzeit (Q-TOF) Massenspektrometrie, Photoionisations-Massenspektrometrie bei atmosphärischem Druck (APPI-MS), Fourier-Transformation Massenspektrometrie (FTMS), Matrix-assistierte-Laserdesorption/Ionisations-Fourier-Transformations-Ionenzyklotronresonanz (MALDI-FT-ICR) Massenspektrometrie, Sekundär-Ionenmassenspektrometrie (SIMS), Polymerasekettenreaktion (PCR) Analyse, quantiative PCR, Echtzeit-PCR, Fluoreszenz-Assay, kolorimetrisches Assay, Chemiluminiszenz-Assay, oder einer Kombination davon verwendet.

7. Verfahren nach einem der Ansprüche 1 bis 5, wobei das Profil ein Nukleinsäureprofil ist, das durch eine Polymerasekettenreaktion (PCR) Analyse, Sequenzierungsanalyse, Analyse durch Elektrophorese, Restriktionsfragmentlängenpolymorphismus (RFLP) Analyse, Northern Blot Analyse, Reverse-Transkriptase-PCR Analyse (RT-PCR), quantitative PCR, quantitative RT-PCR, allelspezifische Oligonukleotidhybridisierungsanalyse, komparative genomische Hybridisierung, Heteroduplex-Mobilitäts-Assay (HMA), Single Strand Conformation Polymorphism-Analyse (SSCP), denaturierende Gradienten-Gelelektrophorese (DGGE), RNAase Mismatch-Analyse, Massenspektrometrie, Massenspektrometrie, Matrixassistierte-Laserdesorption/Ionisations-Flugzeit (MALDI-TOF) Massenspektrometrie, Elektrospray-Ionisation (ESI) Massenspektrometrie, oberflächenverbesserte Laserdesorption/Ionisations-Flugzeit (SELDI-TOF) Massenspektrometrie, Quadrupel-Flugzeit (Q-TOF) Massenspektrometrie, Photoionisations-Massenspektrometrie bei atmosphärischem Druck (APPI-MS), Fourier-Transformation Massenspektrometrie (FTMS), Matrix-assistierte-Laserdesorption/Ionisations-Fourier-Transformations-Ionenzyklotronresonanz (MALDI-FT-ICR) Massenspektrometrie, Sekundär-Ionenmassenspektrometrie (SIMS), Southern-Blot-Analyse, In-Situ-Hybridisierung, Fluoreszenz-In-Situ-Hybridisierung (FISH), chromogene In-Situ-Hybridisierung (CISH), Immunhistochemie (ICH), Mikro-Array, komparative genomische Hybridisierung, Karyotyping, multiplexe ligationsabhängige Sondenamplifikation (MLPA), quantitative Multiplex PCR von kurzen fluoreszierenden Fragmenten (QMPSF), Mikroskopie, methylierungsspezifisches PCR (MSP) Assay, winzige Hpall Fragment-Bereicherung durch ligationsvermitteltes PCR (HELP) Assay, radioaktive Acetat-Kennzeichnungsassays, kolorimetrisches DNS-Acetylierungsassay, Chromatin-Immunopräzipitation kombiniert mit Mikro-Array (ChIP-on-Chip) Assay, Restriction Landmark Genomic Scanning, Methyl-DNS Immunopräzipitation (MeDIP), molekularer Break Light Assay für DNS-Adenin-Methyltransferase-Aktivität, chromatographische Trennung, methylierungsempfindliche Restriktionsenzymanalyse, Oberflächenplasmonresonanz, bisulfit-getriebene Umwandlung von nicht-methyliertem Cytosin zu Uracil, methylbindende PCR-Analyse, oder eine Kombination davon detektiert wird;
wobei die Sequenzierungsanalyse optional aus der Gruppe ausgewählt wird, die aus direkter Sequenzierung, Schrotschuss-Sequenzierung, Dideoxy-Sequenzierung nach Sanger, Sequenzierung von Gesamtgenomen, Sequenzierung durch Hybridisierung, Pyrosequenzierung, Kapillarelektrophorese, Gelelektrophorese, Duplex-Sequenzierung, Zyklus-Sequenzierung, Einzelbasenextensionssequenzierung, Festphasen-Sequenzierung, Hochdurchsatz-Sequenzierung, Massively Parallel Signature Sequenzierung, Emulsion PCR, Sequenzierung durch reversiblen Farbstoffterminator, Paired-End Sequenzierung, Near-Term Sequenzierung, Exonuklease-Sequenzierung, Sequenizerung durch Ligatur, Short-Read Sequenzierung, Einzelmolekül-Sequenzierung, Sequenzierung durch Synthese, Echtzeit-Sequenzierung, Reverse-Terminator Sequenzierung, Nanoporen Sequenzierung, 454 Sequenzierung, Solexa Genome Analyzer Sequenzierung, SOLID® Sequenzierung, MS-PET Sequenzierung, Massenspektrometrie und einer Kombination davon besteht;
oder wobei das Profil ein Proteinprofil ist, das durch einen Immunhistochemie-Assay, Enzyme-Linked Immunosorbent Assay (ELISA), Chromatographie, Flüssigchromatographie, Größenausschluss-Chromatographie, Hochleistungs-Flüssigchromatographie (HPLC), Gaschromatographie, Massenspektrometrie, Tandem-Massenspektrometrie, Matrix-assistierte-Laserdesorption/lonisations-Flugzeit (MALDI-TOF) Massenspektrometrie, Elektrospray-Ionisation (ESI) Massenspektrometrie, oberflächenverbesserte Laserdesorption/Ionisations-Flugzeit (SELDI-TOF) Massenspektrometrie, Quadrupel-Flugzeit (Q-TOF) Massenspektrometrie, Photoionisations-Massenspektrometrie bei atmosphärischem Druck (APPI-MS), Fourier-Transformation Massenspektrometrie (FTMS), Matrix-assistierte-Laserdesorption/Ionisations-Fourier-Transformations-Ionenzyklotronresonanz (MALDI-FT-ICR) Massenspektrometrie, Sekundär-Ionenmassenspektrometrie (SIMS), Radioimmunoassays, Oberflächenplasmonresonanz, mikrofluidische chipbasierte Assays, Western-Blotting-Assay, oder eine Kombination davon detektiert wird.

8. Verfahren nach einem der Ansprüche 1 oder 2, weiter umfassend das Extrahieren zellulärer Inhalte aus den >2n phagozytischen Zellen oder den =2n phagozytischen Zellen vor a).

9. Verfahren nach einem der Ansprüche 1 oder 2, wobei die >2n phagozytischen Zellen oder die =2n phagozytischen Zellen professionelle phagozytische Zellen, nichtprofessionelle phagozytische Zellen oder Mischungen davon sind;
wobei die professionellen phagozytischen Zellen optional Neutrophile, Macrophage, Monozyten, dendritische Zellen, Schaumzellen, Mastzellen, Eosinophile, oder Mischungen davon sind;
wobei die nicht-professionellen phagozytischen Zellen optional Epithelzellen, Endothelzellen, Fibroplaste, mesenchymale Zellen, oder Mischungen davon sind; und
wobei die >2n phagozytischen Zellen oder die =2n phagozytischen Zellen oder die nicht-phagozytischen Zellen optional aus einer Körperflüssigkeitsprobe, Geweben, oder Zellen des Patienten isoliert werden; wobei die Körperflüssigkeitsprobe optional Blut, Urin, Stuhl, Speichel, Lymphflüssigkeit, Zerebrospinalflüssigkeit, Synovialflüssigkeit, Zystenflüssigkeit, Aszites, Pleuralerguss, Flüssigkeit, die von einer schwangeren Frau im ersten Trimester erhalten wird, Flüssigkeit, die von einer schwangeren Frau im zweiten Trimester erhalten wird, Flüssigkeit, die von einer schwangeren Frau im dritten Trimester erhalten wird, Mutterblut, Fruchtwasser, Chorionzottenprobe, Flüssigkeit von einem Präimplantations-Embryo, Urin der Mutter, Speichel der Mutter, Plazentaprobe, fetales Blut, Spülung und Zervikal-Vaginalflüssigkeit, Zwischenzellflüssigkeit oder Augenflüssigkeit isoliert werden.

## Revendications

1. Procédé de surveillance de la progression ou de la régression d'un cancer chez un sujet comprenant :
a) la préparation d'un premier profil d'acide nucléique et/ou de protéine d'un ou de plusieurs marqueurs de cancer par la détection des un ou plusieurs marqueurs parmi une population des cellules phagocytaires >2n du sujet à un premier moment temporel ;
la préparation d'un deuxième profil d'acide nucléique et/ou de protéine d'au moins l'un des un ou de plusieurs marqueurs par la détection d'au moins l'un des un ou plusieurs marqueurs parmi une population des cellules phagocytaires =2n du sujet au premier moment temporel ;
l'identification d'une première différence entre les premier et deuxième profils d'au moins un ou plusieurs desdits marqueurs ;
b) la préparation d'un troisième profil d'acide nucléique et/ou de protéine des un ou plusieurs marqueurs par la détection des un ou plusieurs marqueurs parmi une population des cellules phagocytaires >2n du sujet à un second moment temporel ;
la préparation d'un quatrième profil d'acide nucléique et/ou de protéine d'au moins l'un des un ou de plusieurs marqueurs par la détection d'au moins l'un des un ou plusieurs marqueurs parmi une population des cellules phagocytaires =2n du sujet au second moment temporel ;
l'identification d'une seconde différence entre les troisième et quatrième profils d'au moins un ou plusieurs desdits marqueurs ; et
c) l'identification d'une différence entre la première différence et la seconde différence, dans lequel la différence identifiée indique la progression ou la régression dudit cancer chez le sujet.

2. Procédé selon la revendication 1, dans lequel le profil est un profil d'acide nucléique, un profil de protéine, ou une combinaison de ceux-ci ;
facultativement dans lequel le profil d'acide nucléique est un profil génotypique, un profil de polymorphisme mononucléotidique, un profil de mutation génique, un profil de nombre de copie de gène, un profil de méthylation de l'ADN, un profil d'acétylation de l'ADN, un profil de dosage de chromosome, un profil d'expression génique, ou une combinaison de ceux-ci ; et
facultativement dans lequel le profil de protéine est un profil d'expression de protéine, un profil d'activation de protéine, ou une combinaison de ceux-ci ; et en outre facultativement dans lequel le profil d'activation de protéine comprend la détermination d'un état de phosphorylation, d'un état d'ubiquitination, d'un état de myristoylation, ou d'un état de conformation des un ou plusieurs marqueurs.

3. Procédé selon l'une quelconque des revendications 1 ou 2, dans lequel les un ou plusieurs marqueurs sont des acides nucléiques, des protéines, ou des combinaisons de ceux-ci ;
facultativement dans lequel les acides nucléiques sont des nucléotides, des oligonucléotides, des ADN, des ARN, ou des hybrides ADN-ARN ; et
facultativement dans lequel les protéines sont des acides aminés, des peptides, des enzymes, des antigènes, des anticorps, des cytokines, des lipoprotéines, des glycoprotéines, ou des hormones.

4. Procédé selon l'une quelconque des revendications 2 ou 3, dans lequel les analytes sont des acides nucléiques, des protéines, ou des combinaisons de ceux-ci ;
facultativement dans lequel les acides nucléiques sont des nucléotides, des oligonucléotides, des ADN, des ARN, ou des hybrides ADN-ARN ;
facultativement dans lequel les protéines sont des acides aminés, des peptides, des enzymes, des antigènes, des anticorps, des cytokines, des lipoprotéines, des glycoprotéines, ou des hormones.

5. Procédé selon l'une quelconque des revendications 1 à 4, dans lequel le profil est un profil d'acide nucléique, un profil de protéine, ou une combinaison de ceux-ci ;
facultativement dans lequel le profil d'acide nucléique est un profil génotypique, un profil de polymorphisme mononucléotidique, un profil de mutation génique, un profil de nombre de copie de gène, un profil de méthylation de l'ADN, un profil d'acétylation de l'ADN, un profil de dosage de chromosome, un profil d'expression génique, ou une combinaison de ceux-ci ; et
facultativement dans lequel le profil de protéine est un profil d'expression de protéine, un profil d'activation de protéine, ou une combinaison de ceux-ci ; et en outre facultativement dans lequel le profil d'activation de protéine comprend la détermination d'un état de phosphorylation, d'un état d'ubiquitination, d'un état de myristoylation, ou d'un état de conformation des un ou plusieurs marqueurs.

6. Procédé selon l'une quelconque des revendications 1 à 5, dans lequel le profil est déterminé par un dosage qualitatif, un dosage quantitatif, ou une combinaison de ceux-ci ;
facultativement dans lequel le dosage quantitatif utilise un séquençage, un séquençage direct, un séquençage aléatoire, un séquençage de terminaison didésoxy de Sanger, un séquençage du génome entier, un séquençage par hybridation, un pyroséquençage, une électrophorèse capillaire, une électrophorèse sur gel, un séquençage duplex, un séquençage en cycle, un séquençage par extension d'une seule base, un séquençage en phase solide, un séquençage à haut débit, un séquençage de signature massivement parallèle, une PCR en émulsion, un séquençage par terminateur colorant réversible, un séquençage par extrémités appariées, un séquençage très court terme, un séquençage par exonucléase, un séquençage par ligature, un séquençage à lecture courte, un séquençage d'une seule molécule, un séquençage par synthèse, un séquençage en temps réel, un séquençage par terminateur inverse, un séquençage par nanopores, un séquençage 454, un séquençage par Solexa Genome Analyzer, un séquençage SOLID®, un séquençage MS-PET, une spectrométrie de masse, une spectrométrie de masse à désorption/ionisation laser assistée par matrice à temps de vol (MALDI-TOF), une spectrométrie de masse à ionisation par électropulvérisation (ESI), une spectrométrie de masse à désorption/ionisation laser exaltée de surface à temps de vol (SELDI-TOF), une spectrométrie de masse quadripolaire à temps de vol (Q-TOF), une spectrométrie de masse à photoionisation sous pression atmosphérique (APPI-MS), une spectrométrie de masse à transformée de Fourier (FTMS), une spectrométrie de masse à résonance cyclotronique ionique à désorption/ionisation laser assistée par matrice à transformée de Fourier (MALDI-FT-ICR), une spectrométrie de masse des ions secondaires (SIMS), une analyse par amplification en chaîne par polymérase (PCR), une PCR quantitative, une PCR en temps réel, un dosage par fluorescence, un dosage colorimétrique, un dosage par chimioluminescence, ou une combinaison de ceux-ci.

7. Procédé selon l'une quelconque des revendications 1 à 5, dans lequel le profil est un profil d'acide nucléique détecté par une analyse par amplification en chaîne par polymérase (PCR), une analyse par séquençage, une analyse électrophorétique, une analyse de polymorphisme de longueur de fragment de restriction (RFLP), une analyse par transfert de Northern, une analyse par PCR par transcriptase inverse (RT-PCR), une PCR quantitative, une RT-PCR quantitative, une analyse par hybridation d'oligonucléotide spécifique d'un allèle, une hybridation génomique comparative, un essai de mobilité d'hétéroduplex (HMA), un polymorphisme de conformation des simples brins (SSCP), une électrophorèse sur gel en gradient dénaturant (DGGE), une analyse de mésappariement d'ARNase, une spectrométrie de masse, une spectrométrie de masse, une spectrométrie de masse à désorption/ionisation laser assistée par matrice à temps de vol (MALDI-TOF), une spectrométrie de masse à ionisation par électropulvérisation (ESI), une spectrométrie de masse à désorption/ionisation laser exaltée de surface à temps de vol (SELDI-TOF), une spectrométrie de masse quadripolaire à temps de vol (Q-TOF), une spectrométrie de masse à photoionisation sous pression atmosphérique (APPI-MS), une spectrométrie de masse à transformée de Fourier (FTMS), une spectrométrie de masse à résonance cyclotronique ionique à désorption/ionisation laser assistée par matrice à transformée de Fourier (MALDI-FT-ICR), une spectrométrie de masse des ions secondaires (SIMS), une analyse par transfert de Southern, une hybridation in situ, une hybridation in situ par fluorescence (FISH), une hybridation chromogène in situ (CISH), une immunohistochimie (IHC), un microréseau, une hybridation génomique comparative, un caryotypage, une amplification multiplex de sonde dépendante des ligatures (MLPA), une PCR multiplexe quantitative de fragments fluorescents courts (QMPSF), une microscopie, un dosage par PCR spécifique de la méthylation (MSP), un dosage par enrichissement de petits fragments Hpall par PCR médiée par ligature (HELP), les dosages par marquage par acétate radioactif, un dosage colorimétrique de l'acétylation de l'ADN, une immunoprécipitation de chromatine combinée avec un dosage sur microréseau (ChIP-on-chip), un balayage génomique de repère de restriction, une immunoprécipitation d'ADN méthylé (MeDIP), un dosage par lumière de rupture moléculaire pour l'activité de l'ADN adénine méthyltransférase, une séparation chromatographique, une analyse d'enzyme de restriction sensible à la méthylation, une résonnance plasmonique de surface, une conversion induite par le bisulfite de la cytosine non méthylée en uracile, une analyse par PCR par liaison au méthyle, ou une combinaison de ceux-ci ;
facultativement dans lequel l'analyse par séquençage est sélectionnée dans le groupe consistant en un séquençage direct, un séquençage aléatoire, un séquençage de terminaison didésoxy de Sanger, un séquençage du génome entier, un séquençage par hybridation, un pyroséquençage, une électrophorèse capillaire, une électrophorèse sur gel, un séquençage duplex, un séquençage en cycle, un séquençage par extension d'une seule base, un séquençage en phase solide, un séquençage à haut débit, un séquençage de signature massivement parallèle, une PCR en émulsion, un séquençage par terminateur colorant réversible, un séquençage par extrémités appariées, un séquençage très court terme, un séquençage par exonucléase, un séquençage par ligature, un séquençage à lecture courte, un séquençage d'une seule molécule, un séquençage par synthèse, un séquençage en temps réel, un séquençage par terminateur inverse, un séquençage par nanopores, un séquençage 454, un séquençage par Solexa Genome Analyzer, un séquençage SOLiD®, un séquençage MS-PET, une spectrométrie de masse, et une combinaison de ceux-ci ;
ou dans lequel le profile est un profil de protéine détecté par un dosage par immunohistochimie, un dosage par immunosorbant lié à une enzyme (ELISA), une chromatographie, une chromatographie liquide, une chromatographie d'exclusion stérique, une chromatographie liquide haute performance (CLHP), une chromatographie en phase gazeuse, une spectrométrie de masse, une spectrométrie de masse tandem, une spectrométrie de masse à désorption/ionisation laser assistée par matrice à temps de vol (MALDI-TOF), une spectrométrie de masse à ionisation par électropulvérisation (ESI), une spectrométrie de masse à désorption/ionisation laser exaltée de surface à temps de vol (SELDI-TOF), une spectrométrie de masse quadripolaire à temps de vol (Q-TOF), une spectrométrie de masse à photoionisation sous pression atmosphérique (APPI-MS), une spectrométrie de masse à transformée de Fourier (FTMS), une spectrométrie de masse à résonance cyclotronique ionique à désorption/ionisation laser assistée par matrice à transformée de Fourier (MALDI-FT-ICR), une spectrométrie de masse des ions secondaires (SIMS), un dosage radio-immunologique, une résonance plasmonique de surface, les dosage basés sur les puces microfluidiques, un dosage par transfert de Western, ou une combinaison de ceux-ci.

8. Procédé selon l'une quelconque des revendications 1 ou 2, comprenant en outre l'extraction d'un contenu cellulaire provenant des cellules phagocytaires >2n ou des cellules phagocytaires =2n avant a).

9. Procédé selon l'une quelconque des revendications 1 ou 2, dans lequel les cellules phagocytaires >2n ou les cellules phagocytaires =2n sont des cellules phagocytaires professionnelles, des cellules phagocytaires non professionnelles, ou des mélanges de celles-ci ;
facultativement dans lequel les cellules phagocytaires professionnelles sont des neutrophiles, des macrophages, des monocytes, des cellules dendritiques, des cellules spumeuses, des mastocytes, des éosinophiles, ou des mélanges de ceux-ci ;
facultativement dans lequel les cellules phagocytaires non professionnelles sont des cellules épithéliales, des cellules endothéliales, des fibroblastes, des cellules mésenchymateuses, ou des mélanges de ceux-ci ; et
facultativement dans lequel les cellules phagocytaires >2n ou les cellules phagocytaires =2n ou les cellules non phagocytaires sont isolées à partir d'un échantillon de fluide corporel, de tissus, ou de cellules du sujet ;
facultativement dans lequel l'échantillon de fluide corporel est du sang, de l'urine, des selles, de la salive, du liquide lymphatique, du liquide céphalorachidien, du liquide synovial, du liquide kystique, des ascites, un épanchement pleural, un liquide provenant d'une femme enceinte au cours du premier trimestre, un liquide provenant d'une femme enceinte au cours du deuxième trimestre, un liquide provenant d'une femme enceinte au cours du troisième trimestre, du sang maternel, du liquide amniotique, un échantillon de villosités choriales, un liquide provenant d'un embryon préimplantatoire, de l'urine maternelle, de la salive maternelle, un échantillon placentaire, du sang fœtal, un lavage et un liquide vaginal cervical, un liquide interstitiel, ou un liquide oculaire.
